# EUROPÄISCHE PATENTANMELDUNG

(11) **EP 1 623 987 A1**
(43) Veröffentlichungstag der Anmeldung: **08.02.2006**
(21) Anmeldenummer: 04018272.7
(22) Anmeldetag: 02.08.2004
(51) Int. Cl.: C07D 495/14, C07D 491/14, A61K 31/519, A61P 11/06, A61P 37/08

(54) **Anellierte Pyrido[3',2':4,5]thieno[3,2-d]pyrimidine und Pyrido[3',2':4,5]furo[3,2-d]pyrimidine**

(71) Anmelder: Curacyte Discovery GmbH, 04103 Leipzig (DE)
(72) Erfinder: Reichelt, Claudia, 04299 Leipzig (DE); Schulze, Alexander, 04317 Leipzig (DE); Daghish, Mohammed, 04107 Leipzig (DE); Leistner, Siegfried, 04340 Leipzig (DE)
(74) Vertreter: Schüssler, Andrea

(57) **Zusammenfassung**

Die Erfindung betrifft substituierte anellierte Pyrido[3',2':4,5]thieno[3,2-d]pyrimidine (Y=S) und substituierte anellierte Pyrido[3',2':4,5]furo[3,2-d]pyrimidine (Y=O) der allgemeinen Formel 1 sowie substituierte anellierte Pyrido[3',2':4,5]thieno[3,2-d]pyrimidin-2,4-dione bzw. -4-one (Y=S) und substituierte anellierte Pyrido[3',2':4,5]furo[3,2-d]pyrimidin-2,4-dione bzw. 4-one (Y=O) der allgemeinen Formeln 4a bzw. 4b,

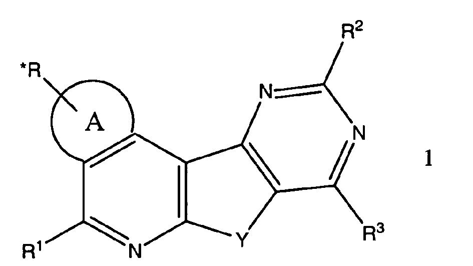

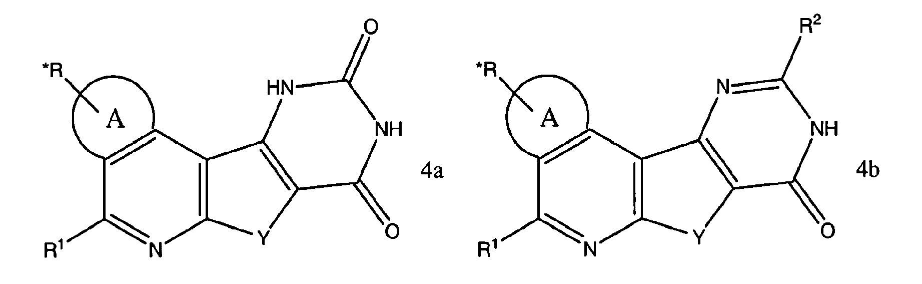

Verfahren zu deren Herstellung, pharmazeutische Zubereitungen, die diese Verbindungen und/oder daraus herstellbare physiologisch verträgliche Salze und/oder deren Solvate enthalten sowie die pharmazeutische Verwendung dieser Verbindungen, deren Salze oder Solvate als Inhibitoren der Phosphodiesterase 4 und/oder TNFα-Freisetzung (in Falle der Verbindungen der allgemeinen Formel 1) sowie als Inhibitoren der TNFα-Freisetzung (im Falle der Verbindungen der allgemeinen Formel 4a und 4b). Die Verbindungen der allgemeinen Formel 1 stellen Wirkstoffe zur Behandlung jener Erkrankungen dar, die insbesondere durch Hemmung der Aktivität von Phosphodiesterase 4 positiv zu beeinflussen sind.

## Beschreibung

### Technisches Gebiet

Die Erfindung betrifft substituierte anellierte Pyrido[3',2':4,5]thieno[3,2-d]pyrimidine (Y=S) und substituierte anellierte Pyrido[3',2':4,5]furo[3,2-d]pyrimidine (Y=O) der allgemeinen Formel 1 sowie substituierte anellierte Pyrido[3',2':4,5]thieno[3,2-d]pyrimidin-2,4-dione bzw. -4-one (Y=S) und substituierte anellierte Pyrido[3',2':4,5]furo[3,2-d]pyrimidin-2,4-dione bzw. 4-one (Y=O) der allgemeinen Formeln 4a bzw. 4b,

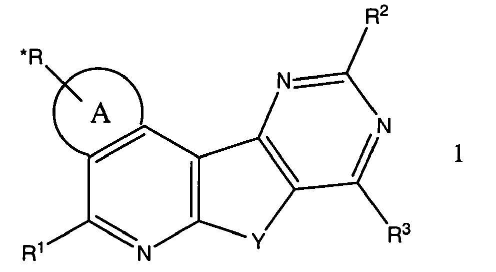

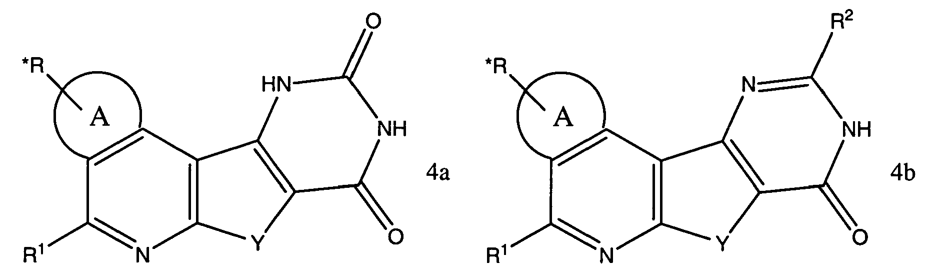

Verfahren zu deren Herstellung, pharmazeutische Zubereitungen, die diese Verbindungen und/oder daraus herstellbare physiologisch verträgliche Salze und/oder deren Solvate enthalten sowie die pharmazeutische Verwendung dieser Verbindungen, deren Salze oder Solvate als Inhibitoren der Phosphodiesterase 4 und/oder TNFα-Freisetzung (in Falle der Verbindungen der allgemeinen Formel 1) sowie als Inhibitoren der TNFα-Freisetzung, z.B. aus Mastzellen, Fibroblasten, Basophilen, Endothelzellen, aktivierten Macrophagen, aktivierten Lymphozyten und Astrozyten (im Gehirn) (im Falle der Verbindungen der allgemeinen Formel 4a und 4b). Die Verbindungen der allgemeinen Formel 1 stellen Wirkstoffe zur Behandlung jener Erkrankungen dar, die insbesondere durch Hemmung der Aktivität von Phosphodiesterase 4, z.B. in Lymphozyten, eosinophilen und basophilen Granulozyten, Makrophagen und Mastzellen, positiv zu beeinflussen sind.

### Stand der Technik

Die zyklischen Nukleotide cAMP und cGMP werden als intrazelluläre sekundäre Botenstoffe (second messengers) zusammengefasst und spielen bei vielfältigsten physiologischen aber auch bei pathophysiologischen Prozessen in lebenden Organismen eine zentrale Rolle. Bei hohen intrazellulären Konzentrationen dieser Nukleotide binden sich diese an spezifische Proteinkinasen, insbesondere an die Proteinkinase A und die Proteinkinase B, und aktivieren diese. Die so aktivierten Proteinkinasen sind nun in der Lage, eine Vielzahl von intrazellulären Proteinen zu phosphorylieren, die dann ihrerseits den zellulären Stoffwechsel und die Antwort dieser Zellen auf innere und äußere Signale maßgeblich beeinflussen.

In der lebenden Zelle wird die Konzentration an cAMP und cGMP hauptsächlich durch die Synthese und den enzymatischen Abbau dieser Nukleotide bestimmt. Die Synthese von cAMP und cGMP erfolgt aus ATP und GTP über die Enzyme Adenylatzyklase und Guanylatzyklase, die durch G-Protein-gekoppelte Rezeptoren in der Zellmembran aktiviert werden. Der Abbau dieser zyklischen Nukleotide erfolgt durch spezifische Phosphodiesterasen in der Zelle.

Die Phosphodiesterasen (PDEs) umfassen eine große Superenzymfamilie mit derzeit 11 bekannten Typen, die ihrerseits in zahlreiche Subtypen (Isoformen) und noch wesentlich mehr Splicevarianten unterteilt werden. Diese Isoformen unterscheiden sich in ihrer Substratspezifität, Enzymkinetik, Gewebespezifität, Sensitivität gegenüber Inhibitoren und Aktivatoren sowie in ihrer intrazellulären Kompartmentierung *(Giernbycz MA. Phosphodiesterase 4 inhibitors and the treatment of asthma: where are we now and where do we go from here? Drugs [2000]*; *59, 193-212).*

Seit 1990 ist ein wachsendes Interesse der pharmazeutischen Industrie zu registrieren, selektive PDE Inhibitoren zu identifizieren und als Wirkstoffkandidaten zur Behandlung von z.B. chronischen Atemwegserkrankungen zu entwickeln.

Die cAMP spezifische PDE-4 stellt nach heutigem Wissensstand das wichtigste Isoenzym von immunkompetenten Zellen und von glatten Muskelzellen des Lungengewebes dar. Die PDE-4 Familie umfasst derzeit vier Gene mit den Genprodukten PDE-4A, PDE-4B, PDE-4C und PDE-4D. Erstmals wurden PDE-4 spezifische Isotypen von Nemoz et al. beschrieben *(Nemoz G, Prigent AF, Moueqqit M, Fougier S, Macovschi O, Pacheco H. Selective inhibition of one of the cyclic AMP phosphodiesterases from rat brain by the neurotropic compound rolipram. Biochem Pharmacol [1985]; 34, 2997-3000).* Alle Subtypen setzen sich aus einer konservierten katalytischen Domäne mit einer Länge von ca. 270 Aminosäuren und zwei in der Länge variierenden zusätzlichen Regionen (UCR1 und UCR2; upstream conserved region) innerhalb des N-Terminus des Moleküls zusammen *(Engels P, Fichtel K, Lubbert H. Expression and regulation of human and rat phosphodiesterase IV. FEBS Letters [1994]; 350, 291-295).* Eine weitere Diversivität in der Expression der PDE-4 Isoformen resultiert durch alternative Splicevarianten und unterschiedliches posttranslationales Prozessing *(Beavo JA, Conti M, Heaslip RJ. Multiple cyclic nucleotide phosphodiesterases. Mol Pharmakol [1994]; 46, 399-405).* Diese vier Subtypen werden in unterschiedlichen Geweben und Zellen differentiell exprimiert. Die PDE-4A bis -4D sind in den meisten immunkompetenten Zellen und in Entzündungszellen vorhanden. Mit Ausnahme der PDE-4B sind die anderen PDE-4 Typen auch in den Epithelzellen der Luftwege nachweisbar. Im menschlichen Gehirn ist gleichfalls eine differentielle Expression der vier Subtypen in Abhängigkeit von bestimmten anatomischen Regionen beschrieben.

In den vergangenen Jahren haben zahlreiche präklinische und klinische Studien die Modulation von Entzündungs- und Immunkompetenten Zellen durch selektive PDE-4 Inhibitoren belegen können und führten damit zu der wissenschaftlich abgesicherten Auffassung über das hohe therapeutische Potential von spezifischen PDE-4 Inhibitoren bei ganz unterschiedlichen entzündlichen Erkrankungen, wie z.B. chronisch obstruktiven Lungenerkrankungen (COPD), Asthma bronchiale, atopischer Dermatitis, Rheumatoider Arthritis (RA), Multiple Sklerose (MS) und zahlreichen neurologischen Erkrankungen *(Doherty AM. Phosphodiesterase 4 inhibitors* as *novel anti-inflammatory agents. Curr Opin Chem Biol [1999]; 3, 466-473; Essayan DM. Cyclic nucleotide phosphodiesterase (PDE) inhibitors and immunomodulation. Biochem Pharmacol [1999]; 57, 565-573; Dal Piaz V, Giovannoni MP. Phosphodiesterase 4 inhibitors, structurally unrelated to Rolipram, as promising agents for treatment of asthma and other pathologies. Eur J Med Chem [2000]; 35, 463-480).*

Eine zentrale Eigenschaft von selektiven PDE-4 Inhibitoren ist die Hemmung der Freisetzung des wichtigen pro-inflammatorischen Zytokins TNFα aus Entzündungszellen (Makrophagen, Mastzellen, T-Lymphozyten, basophilen und eosinophilen Granulozyten) aber auch aus Fibroblasten, Endothelzellen und Astrozyten. TNFα wiederum ist selbst in der Lage, weitere proi-nflammatorische Zytokine wie den granulocyte-macrophage colony-stimulating factor (GM-CSF) und Interleukine, besonders IL-1 und IL-8, zu stimulieren. Desweiteren aktiviert TNFα neutrophile und eosinophile Granulozyten, Fibroblasten und Endothelzellen, die ihrerseits pro-inflammatorische Zytokine und Proteasen, hauptsächlich MMP's, sezernieren. So stellen auch alle Krankheiten, bei denen TNFα eine zentrale Rolle im Erkrankungsprozess spielt, ein Target für PDE-4 Inhibitoren dar.

In eosinophilen Granulozyten konnten sowohl PDE-3 als auch PDE-4A, B, D, jedoch keine PDE-4C Subtypen nachgewiesen werden. Diese Zellen spielen eine bedeutende Rolle in der Pathophysiologie des Asthmas und sind deshalb auch hinsichtlich der PDE-4 Hemmung intensiv untersucht worden. Die selektive PDE-4 Hemmung bewirkt eine Verminderung der Freisetzung von reaktiven Sauerstoffradikalen und von Leukotrien C₄ *(Sturton RG, Butt NM, Palfai SP, Tudhope SR, Abram TS, Fisher R, Braunlich G, Es-Sayed M. Am J Respir Crit Care Med [2000]; 161, A200).* Die Synthese des eosinophil-derived neurotoxin (EDN), der Komplementkomponente C5ₐ und des platelet activating factor (PAF) wird gleichfalls durch selektive Inhibition der PDE-4 vermindert *(Hatzelmann A, Tenor H, Schudt C. Differential effects of non-selective and selective phosphodiesterase inhibitors on human eosinophil functions. BrJ Pharmacol. [1995];114, 821-831).* PDE-4 Inhibitoren blockieren, untersucht in verschiedensten Tiermodellen für Asthma, signifikant das Einwandern von eosinophilen Granulozyten, hemmen die Akkumulation der Zytokine IL-4 und IL-5 in der broncho-alveolären Lavage (BAL) und verbessern die Funktion der Luftwege *(Kanehiro A, Ikemura T, Makela MJ, Lahn M, Joetham A, Dakhama A, Gelfand EW. Inhibition of phosphodiesterase 4 attenuates airway hyperresponsiveness and airway inflammation in a model of secondary allergen challenge. Am J Respir Crit Care Med. [2001];163, 173-184).*

Neutrophile Granulozyten spielen eine Schlüsselrolle bei entzündlichen Erkrankungen. Sie akkumulieren sehr schnell im entzündlichen Gewebe und sezernieren eine Vielzahl biologisch aktiver Mediatoren und Enzyme. In diesen Zellen wird als cAMP-abbauendes Enzym hauptsächlich die PDE-4 nachgewiesen *( Wang P, Wu P, Ohleth KM, Egan RW, Billah MM. Phosphodiesterase 4B2 is the predominant phosphodiesterase species and undergoes differential regulation of gene expression in human monocytes and neutrophils. Mol Pharmacol. [1999]; 56,170-174*). PDE-4 Inhibitoren suprimieren eine Vielzahl von Abwehrfunktionen dieser Zellen, wie Superoxid-Bildung, Degranulierung, IL-8-Freisetzung, Adhesionsmolekül-Expression, eine stark verminderte Expression der humanen Lungen-Elastase, der MMP-9 und der Leukotrien B₄ Synthese *(Barnette MS, Christensen SB, Essayan DM, Grous M, Prabhakar U, Rush JA, Kagey-Sobotka A, Torphy TJ. SB 207499 (Ariflo), a potent and selective second-generation phosphodiesterase 4 inhibitor: in vitro anti-inflammatory actions. J Pharmacol Exp Ther. [1998]; 284, 420-426; Berends C, Dijkhuizen B, de Monchy JG, Dubois AE, Gerritsen J, Kauffman HF. Inhibition of PAF-induced expression of CD11b and shedding of L-selectin on human neutrophils and eosinophils by the type IV selective PDE inhibitor, rolipram. Eur Respir J. [1997]; 10, 1000-1007*)*.* Eine pathophysiologische Hauptkonsequenz dieser Zellfunktionsänderung durch PDE-4 Inhibitoren ist in deren stark verminderter Adhäsion und Chemotaxis in Richtung des Entzündungsherdes zu sehen, es kommt somit nicht zum Einwandern der Entzündungszellen.

PDE-4 Inhibitoren blockieren in vivo in zahlreichen Tiermodellen die Neutrophilie in der BAL und damit einen wesentlichen pathologischen Entzündungsweg *(Spond J, Chapman R, Fine J, Jones H, Kreutner W, Kung TT, Minnicozzi M. Comparison of PDE 4 inhibitors, rolipram and SB 207499 (ariflo), in a rat model of pulmonary neutrophilia. Pulm Pharmacol Ther[2001], 14, 157-164).*

Das gleichfalls verstärktes Einwandern und die Akkumulation von Monozyten in das entzündliche Lungengewebe ist ein typischer pathophysiologischer Regulationsmechanismus bei diesen Erkrankungen. Als Leitphänomen kann hierbei die Freisetzung von pro-inflammatorischen Zytokinen, besonders von TNFα und GM-CSF und zahlreicher proteolytischer Enzyme angesehen werden. Selektive PDE-4 Inhibitoren blockieren die Synthese und Freisetzung von TNFα, GM-CSF und Proteasen und stimulieren die PGE₂ Produktion. Überraschenderweise wird die Synthese des anti-inflammatorische Zytokin IL-10 durch selektive PDE-4 Inhibitoren stark stimuliert *(Suda Y, Tamura G, Ohno I, Maeda K, Liu Y, Yamauchi K, Kurimoto F, Shirato K. Effects of phosphodiesterase inhibitors on secretions of human monokines. Allergol Int [1998]; 47, 219-224*)*.*

Die im entzündlichen Lungengewebe akkumulierten T-Lymphozyten sezernieren hauptsächlich pro-inflammatorische Zytokine wie IL-2, IL-4, IL-5, IL-13, TNFα, IFN_{γ} und GM-CSF, deren Synthese durch PDE-4 Inhibitoren sehr effektiv gehemmt wird.

Die besonders bei Asthma eingewanderten B-Lymphozyten werden durch spezifische PDE-4 Inhibitoren in ihrer IgE Synthese gehemmt und damit direkt Einfluss auf den IgE-vermittelten allergischen Pathogeneseweg *(Coqueret O, Boichot E, Lagente V. Selective type IV phosphodiesterase inhibitors prevent IL-4-induced IgE production by human peripheral blood mononuclear cells. Clin Exp Allergy [1997]; 27, 816-823).*

Die Epithelzellen der Luftwege sind gleichfalls stark in die entzündlichen Reaktionen bei COPD und Asthma eingebunden. Bei ihrer Aktivierung setzen sie eine Vielzahl von biologisch hochaktiven Substanzen, wie z.B. Metabolite der Arachidonsäure und proinflammatorische Zytokine, wie das TNFα und GM-CSF, frei *(Chipappara G, Merendino AM, Chimenti L, Rilcobono L, Mirabella F, La Rocca AM, Weck PK, Bonsignore G, Vignola AM. In vitro and ex vivo effects of the phosphodiesterase 4 inhibitors. Am J Resp Crit Cara Med [2001]; 163, A278).* In vivo zeigen PDE-4 Inhibitoren durch eine signifikante Hemmung der Synthese und Freisetzung des proinflammatorischen Enzyms MMP-9, einen starken protektiven Effekt auf Epithelzellen der Luftwege, sowohl in Tiermodellen als auch in klinischen Studien *(Rabinovici R, Feuerstein G, Abdullah F, Whiteford M, Borboroglu P, Sheikh E, Phillip DR, Ovadia P, Bobroski L, Bagasra O, Neville LF. Locally produced tumor necrosis factor-alpha mediates interleukin-2-induced lung injury. Circ Res (1996); 78, 329-236; Ortiz JL, Cortijo J, Valles JM, Bou J, Morcillo EJ. Rolipram inhibits airway microvascular leakage induced by platelet-activating factor, histamine and bradykinin in guinea-pigs. J Pharm Pharmacol (1993); 45, 1090-1092).*

Ein erhöhter intrazellulärer cAMP Spiegel senkt die Aktivität von Immun- und Entzündungszellen sowohl in vitro als auch in vivo, wirkt protektiv auf Epithel- und Endothelzellen und vermittelt eine Relaxation der glatten Muskelzellen im Lungengewebe. Diese pharmakologischen Eigenschaften belegen nachhaltig die Bedeutung von PDE Hemmung als relevantes und abgesichertes Target für die therapeutische Intervention bei chronischen entzündlichen Lungenerkrankungen wie COPD und Asthma *(Barnes PJ, Shapiro SD, Pauwels RA. Chronic obstructive pulmonary disease: molecular and cellular mechanisms. Eur Respir J (2003); 22, 672-88).*

Unter COPD wird eine Gruppe von chronisch progressiven, entzündlichen Lungenerkrankungen zusammengefasst, die hauptsächlich durch ein Einwandern von neutrophilen Granulozyten und weiterer immunkompetenter Zellen in das Lungengewebe charakterisiert ist. Hauptsymptome sind hierbei chronischer Husten und Auswurf und die damit verbundene progrediente und irreversible Verschlechterung der Lungenfunktion bis hin zur Maximalvariante, dem Lungenemphysem. Die Erkrankung verläuft schubförmig und ist häufig mit bakteriellen Sekundärinfektionen der Lunge verbunden. Wichtigste Risikofaktoren sind das Rauchen und die Umweltverschmutzung. Durch die teilweise extreme Kurzatmigkeit und die geringe Belastbarkeit der Patienten auf Grund eines pulmonalen Rückstaus und der daraus resultierenden kardialen Probleme ist die Lebensqualität und Lebenserwartung der Patienten stark eingeschränkt. Weltweit leiden ca. 600 Mill. an dieser Erkrankung und ist laut WHO sechsthäufigste Erkrankung weltweit, die auch die vierthäufigste Todesursache ist. Mit einer jährlichen Wachstumsrate von 12% (Visiongain, 2004), wird sie global in den nächsten 20 Jahren die dritthäufigste Todesursache sein. Analysten schätzen deshalb ein, dass es sich bei dieser Erkrankungsgruppe um den mit am schnellsten wachsenden globalen therapeutischen Markt handelt. Die gegenwärtige Standardtherapie setzt sich aus der Gabe von β₂-Agonisten, muscarinergen Antagonisten, Kortikosteroiden und Antihistaminika zusammen. Diese Behandlungsstrategien sind jedoch nur symptomatischer Natur, ohne kausal in den progressiven Verlauf der Erkrankung einzugreifen. Es besteht deshalb seit Jahren die berechtigte Forderung mittels neuer Therapieansätze kausal in den Krankheitsprozess einzugreifen und damit insbesondere den fortschreitenden Charakter von COPD zu bekämpfen.

Beim Asthma bronchiale handelt es sich um eine anfallsweise, nichtinfektiöse und meist allergisch ausgelöste Lungenerkrankung, die hauptsächlich durch ein Einwandern von eosinophilen Granulozyten und weiteren immunkompetenten Zellen in das Lungengewebe und durch eine temporäre Bronchokonstriktion charakterisiert ist. Nach längerer Krankheitsdauer tritt vielfach eine chronische Bronchitis mit einem Lungenemphysem und Bronchiektasien bis hin zum Cor pulmonale auf. Die Lebensqualität der Patienten ist durch die Atemnot bis hin zu Dyspnoe Attacken, die erhebliche Schleimsekretion und starke Angstgefühle merklich eingeschränkt. In den Industrieländern leiden ca. 5% der erwachsenen Bevölkerung und nahezu 10% der Kinder an dieser Erkrankung. Weltweit geht man von einer Prävalenz von 155 Mill. Erkrankten bei einer jährlichen Steigerungsrate von 10 - 15% (aus Lead Discovery, 2003). Die seit 25 Jahren übliche Standardtherapie mit ß₂-Adeno-rezeptor-Agonisten (Bronchodilatation) und Kortikosteroide (antientzündlich) zeigt neben einem guten therapeutischen Profil auch teilweise erhebliche unerwünschte Nebenwirkungen, besonders im Kindesalter (bekannte Glukokortikoid-Nebenwirkungen und Tachykardie, Herzklopfen, Kopfschmerz bei β₂-Adenorezeptor Agonisten). Es besteht auch bei diesem Krankheitsbild die Forderung nach neuen wirksameren Therapiestrategien, die nicht nur symptomatisch, sondern kausal in den Krankheitsprozess eingreifen.

Selektive PDE-4 Inhibitoren sollten daher zusammengefasst zumindest folgende invivo Wirkungen haben:
1. effektive Hemmung der TNFα-Synthese und -Freisetzung in Blutzellen;
2. signifikante Verminderung der TNFα Konzentration in der Broncho-alveolären Lavageflüssigkeit (BAL);
3. signifikante Verminderung von IL-4 und IL-5 in der BAL Flüssigkeit;
4. signifikante Hemmung des Einwanderns von eosinophilen Granulozyten in die Lunge;
5. signifikante Hemmung des Einwanderns von neutrophilen Granulozyten in die Lunge;
6. Verhinderung des "oxidativen Burst";
7. Verhinderung der Bronchokonstriktion;
8. Verhinderung der Ausbildung von Lungenödemen (Emphysem);
9. Verhinderung der Proliferation und Hyperplasie von Zellen der Luftwege;
10. signifikante Hemmung der MMP-9 Aktivität in der BAL Flüssigkeit;
11. signifikante Hemmung der TGF-β Aktivität in der BAL Flüssigkeit.

In neuerer Zeit belegen experimentelle Daten auch den erfolgversprechenden Einsatz von PDE-4 Inhibitoren als immunmodulatorische Wirkstoffe. Das therapeutische Potential von PDE-4 Inhibitoren wurde erfolgreich in Tiermodellen für die atopische Dermatitis *(Hanifin JM, Chan SC, Cheng JB, Tofte SJ, Henderson WR Jr, Kirby DS, Weiner ES. Type 4 phosphodiesterase inhibitors have clinical and in vitro anti-inflammatory effects in atopic dermatitis. J Invest Dermatol (1996); 107, 51-*56), die Rheumatoide Arthritis *(Laemont KD, Schaefer CJ, Juneau PL, Schrier DJ. Effects of the phosphodiesterase inhibitor rolipram on streptococcal cell wall-induced arthritis in rats. Int J Immunopharmacol (1999); 21, 711-725),* Colitis ulcerosa *(Hartmann G, Bidlingmaier C, Siegmund B, Albrich S, Schulze J, Tschoep K, Eigler A, Lehr HA, Endres S. Specific type IV phosphodiesterase inhibitor rolipram mitigates experimental colitis in mice. J Pharmacol Exp Ther (2000); 292, 22-30),* Multiple Sklerose *(Dinter H, Onuffer J, Faulds D, Perez HD. Phosphodiesterase type IV inhibitors in the treatment of multiple sclerosis. J Mol Med (1997); 75, 95-102),* Crohn's Disease *(Prehn JL, Landers C, Muller GW, Man HW, Stirling D1, Targan SR. Potent inhibition of cytokine production from intestinal lamina propria T cells by phosphodiesterase-4 inhibitory thalidomide analogues. J Clin Immunol (2001); 21,* 357-364), Entzündungsschmerz *(Cunha FQ, Teixeira MM, Ferreira SH. Pharmacological modulation of secondary mediator systems--cyclic AMP and cyclic GMP--on inflammatory hyperalgesia. Br J Pharmacol (1999);127, 671-678)*, Septischer Schock *(Cardelus I, Gras J, Jauregui J, Lienas J, Palacios JM. Inhibition of lipopolysaccharide-induced bowel erythrocyte extravasation in rats, and of mesenteric hypoperfusion in dogs, by phosphodiesterase inhibitors. Eur J Pharmacol* (1996); *299*, *153-159)*, Leishmaniose *(Rascon A. Cyclic nucleotide phosphodiesterases: diversity, classification, structure and function. Acta Cient Venez* (*1997*); *48, 145-153*) und HIV Infektionen (Sun *Y, Li L, Lau F, Beavo JA, Clark EA. Infection of CD4+ memory T cells by HIV-1 requires expression of phosphodiesterase 4. J Immunol (2000);* 165, *1755-1761)* nachgewiesen.

Klinisch zeigten die meisten der bisher getesteten PDE-4 Inhibitoren, mit Rolipram als Leitstruktur, unerwünschte Arzneimittelwirkungen (UAW) und fehlende in vivo Wirksamkeit, die ihren klinischen Einsatz bisher limitierten. Dazu zählen emetische Wirkungen wie Übelkeit und Erbrechen, aber auch gastro-intestinale Beschwerden, kardiovaskuläre und zentrale Nebenwirkungen *(Zeller E, Stief HJ, Pflug B, Sastre-y-Hernandez M. Results of a phase II study of the antidepressant effect of rolipram. Pharmacopsychiatry (1984); 17, 188-190; Puurunen J, Lucke C, Schwabe U. Effect of the phosphodiesterase inhibitor 4-(3-cyclopentyloxy-4-methoxyphenyl)-2-pyrrolidone (ZK 62711) on gastric secretion and gastric mucosal cyclic AMP. Naunyn Schmiedebergs Arch Pharmacol (1978); 304, 69-75; Nicholson CD. Cyclic nucleotide phosphodiesterase isoenzymes and asthma--outstanding issues. Agents Actions Suppl (1993); 43, 3-12).*

Ausgehend von Rolipram wurden in der Folgezeit zahlreiche strukturbasierte Abwandlungen entwickelt, die aber alle ein ähnliches Nebenwirkungsprofil aufweisen *(Schneider HH, Schmiechen R, Brezinski M*, *Seidler J. Stereospecific binding of the antidepressant rolipram to brain protein structures. Eur J Pharmacol* (*1986*); *127*, *105-115*). Die heute am weitesten entwickelten PDE-4 Inhibitoren sind Cilomilast (Fa. Glaxo-SmithKline) *(Compton CH, Gubb J, Nieman R, Edelson J, Amit O, Bakst* A, *Ayres JG, Creemers JP, Schultze-Werninghaus* G, *Brambilla C, Barnes NC; International Study Group. Cilomilast,* a *selective phosphodiesterase-4 inhibitor for treatment of patients with chronic obstructive pulmonary disease:* a *randomised, dose-ranging study. Lancet (2001); 358, 265-270; Compton* C, *Edelson JD, Cedar E et al. Cilomilast (Ariflo) 15mg bid safety in* a *six month clinical trial program.* Am *J Resp Crit Care Med (2001); 163, A909; Zussman BD, Benincosa LJ, Webber DM, Clark DJ, Cowley H, Kelly J, Murdoch RD, Upward J, Wyld P, Port A, Fuder H. An overview of the pharmacokinetics of cilomilast (Ariflo), a new, orally active phosphodiesterase 4 inhibitor, in healthy young and elderly volunteers. J Clin Pharmacol (2001); 41, 950-958; Giembycz MA. Cilomilast: a second generation phosphodiesterase 4 inhibitor for asthma and chronic obstructive pulmonary disease. Expert Opin Investig Drugs (2001); 10, 1361-1379)* und Roflumilast (Fa. Altana) ( *Schmidt BM, Kusma M, Feuring M, Timmer WE, Neuhauser M, Bethke T, Stuck BA, Hormann K, Wehling M. The phosphodiesterase 4 inhibitor roflumilast is effective in the treatment of allergic rhinitis. J Allergy Clin Immunol (2001); 108, 530-536; SCRIP 2644, p.25),* die strukturell große Ähnlichkeiten zu Rolipram aufweisen, aber offensichtlich ein besseres Wirkprofil besitzen. Roflumilast ist im Vergleich zu Cilomilast mit einem IC₅₀ Wert von 0,8 nM ein wesentlich potenterer PDE-4 Inhibitor und zeigt auch in zahlreichen in vitro und in vivo Untersuchungen eine stärkere anti-inflammatorische Aktivität *(Hatzelmann A, Schudt C. Anti-inflammatory and immunomodulatory potential of the novel PDE4 inhibitor roflumilast in vitro. J Pharmacol Exp Ther (2001); 297, 267-79).* Beide Wirkstoffe haben trotz eines Nebenwirkungsprofils erfolgreich die klinische Studie Phase III für COPD und Asthma abgeschlossen, sind jedoch noch nicht von den zuständigen Behörden zugelassen worden.

Die bisher entwickelten PDE-4 Inhibitoren, die zum Teil ein umfangreiches Nebenwirkungsprofil aufweisen, lassen sich hauptsächlich in folgende chemische Hauptklassen untergliedern:
1. Catecholetherverbindungen mit struktureller Ähnlichkeit zu Rolipram, Cilomilast und Roflumilast,
2. Chinazolindione mit struktureller Ähnlichkeit zu Nitraquazone,
3. Xanthin-Derivate mit struktureller Ähnlichkeit zu Theophyllin und
4. Benzofuran-Derivate.

Das Zytokin Tumour necrosis factor (TNFα) ist eines von heute 17 bekannten Mitgliedern einer strukturell sehr ähnlichen Proteinfamilie. Seinen Namen verdankt es der Fähigkeit, eine Nekrose von transplantierten Tumorzellen im Mausmodell zu triggern. Neben seiner Apoptose-induzierenden Wirkung wurde sehr schnell erkannt, dass TNFα auch ganz maßgeblich in die Regulation der Entzündungsantwort und der Immunantwort eingebunden ist. Eine Überproduktion von TNFα oder die Aktivierung der TNFα-vermittelten Signalkaskaden spielen in der Pathogenese einer Vielzahl von Erkrankungen, wie z.B. Sepsis, cerebrale Form der Malaria, neurodegenarativen Erkrankungen wie z.B. Mb. Alzheimer, Mb. Parkinson, bei Diabetes mellitus, COPD/Asthma, Tumorerkrankungen und hier insbesondere Tumoren des blutbildenden Systems wie z.B. Leukämien und Lymphome, virale Erkrankungen und hier insbesondere retrovirale Erkrankungen wie z.B. das erworbene Immundefizienz Syndrom (AIDS), Guillain-Barre Syndrom, Rhinitis allergica, allergische Konjunktivitis, systemische Sklerodermie, Graft versus host disease (GvHD), Systemischer Lupus Erythematodes (SLE), Osteoporosis, Toxisches Schocksyndrom, Akute Glomerulonephritis, akute und chronische Schmerzen, Arteriosklerose, Herzinfarkt, Schlaganfall, bei Sarkoidose, Multiple Sklerose, Rheumatoid Arthritis (RA), Osteoarthritis, Colitis ulcerosa, Vasculitis, Uveitis, Mb. Crohn, Mb. Behcet, Myastenia Gravis und chronisch entzündlichen Hauterkrankungen wie Psoriasis, atopische Dermatitis, Ekzeme und Alopecie, eine zentrale Rolle *(Chen G, Goeddel DV (2002 TNF-R1 signaling: a beautiful pathway. Science 296 1634-1635; Ware CF (2003) The TNF superfamily*. *Cytokine & Growth Factor reviews 14 181-184; Dempsey PW (2003) The signaling adaptors and pathways activated by TNF superfamily*. *Cytokine & Growth Factor reviews 14 193-209* ).

Bei TNFα handelt es sich um eines der wichtigsten pro-inflammatorischen Zytokine, der in die Pathogenese fast aller chronisch entzündlichen Erkrankungen maßgeblich eingebunden ist. TNFα, welches auch als Chachektin, Makrophagen-Cytotoxin (MCT), E tumor necrosis factor-a und als macrophage cytotoxic factor (MCF) beschrieben wurde, wird von verschiedensten Zellen nach Stimulation mit Lipopolysaccharid (LPS), Interferronen (IFN's), IL-2, Bradykinin, GM-CSF, Antigen-Antikörper-Komplexen, Substanz P und zahlreichen weiteren biologisch aktiven Verbindungen synthetisiert und sezerniert. TNFα wird unter physiologischen Bedingungen hauptsächlich von Makrophagen, T-Lymphozyten, Mikrogliazellen und NL-Zellen gebildet. Stimulierte und somit aktivierte Fibroblasten, glatte Muskelzellen, Astrozyten, Keratinozyten, Endothelzellen und Lungen-Epithelzellen sezernieren gleichfalls TNFα.

Humanes TNFα ist ein 17 kDa großes Protein, welches aus 157 Aminosäuren besteht und zu Dimeren und Trimeren assoziiert. Es existiert eine weitere höhermolekulare Variante dieses Moleküls mit einer Molmasse von 26 kDa, das als Transmembranprotein in der Zellmembran verankert ist. Man geht heute davon aus, dass zunächst die höhermolekulare Transmembranform synthetisiert wird, deren extrazelluläre Domäne durch das TNFα converting enzyme (TACE) abgespalten wird. Das lösliche TNFα zirkuliert als ein Homotrimer und bindet sich an seine spezifischen Rezeptoren an Zelloberflächen. Die Bindung von TNFα an seine Rezeptoren (TNFR1, TNFR2) bewirkt bei diesen eine konformative Änderung und Dimerisierung bzw. Clusterung der Rezeptoren, welche über eine Signalkaskade den biologischen Effekt von TNFα vermitteln. In zahlreichen Untersuchungen konnte gezeigt werden, dass über die Bindung von TNFα an den TNFR1 die meisten biologischen Effekte realisiert werden. Dies beinhaltet die Induktion der Apoptose über eine Aktivierung der Caspase 8 und nachfolgender Aktivierung der Caspasen 3,6 und 7, die dann unmittelbar zur Apoptose der Zelle führen.

Ein weiterer wichtiger Signalweg durch TNFα ist die Aktivierung von zwei wichtigen Transkriptionsfaktoren, dem nuclear factor-kappaB (NF-_{κ}B) und c-Jun. Diese beiden Transkriptionsfaktoren spielen eine außerordentlich wichtige Rolle in der Regulation der Genexpression bei der Zelldifferenzierung, dem Zellwachstum, bei der Immun- und Entzündungsantwort, bei Zellstressregulationsvorgängen und bei der Tumorgenese. NF-_{κ}B reguliert unter anderem die Gene für IL-1α, IL-1β, IL-2, IL-3, IL-6, IL-8, IL-12, TNFα, LT-α, IFN-α/β, G-CSF, M-CSF, GM-CSF, für den Zytokinrezeptor IL-2Rα, für die Adhäsionsmoleküle ICAM-1, VCAM-1, MAdCAM, E-Selektin, für die immunregulatorischen Moleküle leichte Kette des Igy, MHC Class I und II, TCRα und ß, ß₂ Mikroglobulin, TAP1, iNOS und für die Akute Phase Proteine SAA, α₁-saures Glycoprotein und TSG-14/PTX3.

Über die tatsächliche physiologische Bedeutung der Bindung von TNFα an den TNFR2 existieren heute noch erhebliche Widersprüche. Deshalb ist die Aufdeckung der genauen molekularen Signaltransduktionsabläufe noch Gegenstand der Grundlagenforschung. Mehrheitlich geht man heute davon aus, dass die Bindung von TNFα an den TNFR2 auch die Mitogen-aktivierten Proteinkinase Kinasen (MAPKK) aktiviert, im speziellen die MEKK1 und die ASK1, die über eine Aktivierungskaskade zur Aktivierung der c-Jun Kinase (JNK) und damit zu einer Aktivierung des Transkriptionsfaktors c-Jun führt. In diesen Regulationsweg ist auch die Aktivierung der p38 Kinase eingebunden, die zur Aktivierung von p38 führt. Die Aktivierung von p38 ist essentiell für die Produktion der pro-inflammatorischen Zytokine IL-1β, TNFα und IL-6 und ist darüber hinaus auch verantwortlich für die Induktion und Expression der mit chronischen Entzündung vergesellschafteten Enzyme COX-2 und iNOS *(Ono K, Han J (2000) The p38 signal transduction pathway: activation and function. Cell Signal 12 1-13).* Über weitere Aktivierungswege werden auch die wichtigen Transkriptionsfaktoren activatingtranscription factor 2 (ATF2) und das Aktivatorprotein-1 (AP-1) induziert, welche unmittelbar stimulierenden Einfluss auf die Expression pro-inflammatorischer Moleküle wie E-Selectin, RANTES, IL-12, IL-6 und IL-8 ausüben *(Guicciardi ME, Gores GJ (2003)J Clin Invest 111 1813-1815*)*.*

Die biologische Bedeutung von TNF erkannten erstmals 1969 GRANGER et al. *(Granger GA, Shacks SJ, Williams TW, Kolb WP (1969) Lymphocyte in vitro cytotoxicity: specific release of lymphotoxin-like materials from tuberculin-sensitive lymphoid cells. Nature 221 1155-1157)* die zeigen konnten, dass ein von Lymphozyten und Makrophagen sezerniertes Protein (Lymphotoxin) zur Lyse von Zellen, insbesondere von Tumorzellen, führt. 1984 konnten GRAY et al. *(Gray PW, Aggarwal BB, Benton CV, Briingman TS, Henzel WJ, Jarrett JA, Leung DW, Maffatt B, Ng P, Svedersky LP et al. (1984) Cloning and expression of cDNA for human lymphotoxin, a lymphokine with tumor necrosis activity. Nature 312 721-724)* und PENNICA et al. *(Pennica D, Nedwin GE, Hayflick JS, Seeburg PH, Deyrynck R, Palladino MA, Kohr WJ, Aggarwal BB, Goeddel DV (1984) Human tumor necrosis factor: precursor structure, expression and homology to lymphotoxin. Natur 312 724-729)* die cDNA für TNFα klonieren und das Protein exprimieren.

Die biologische Aktivität von TNFα wird hauptsächlich über zwei spezifische Rezeptortypen (TNFR1, TNFR2) vermittelt, die sich transmembran und mit einem extra- und intrazellulären Anteil auf einer Vielzahl Zellen des menschlichen Körpers befinden.

TNFα besitzt ein sehr breites Spektrum an biologischen Aktivitäten und reguliert fast alle Zellen. Er ist aus heutiger Sicht ein wesentlicher Mediator bei Entzündungs- und Immunreaktionen, aber auch bei der Apoptose, der Zelldifferenzierung, bei der Induktion von Fieber und zahlreichen weiteren pathophysiologischen Regulationsprozessen.

Eine zentrale Stellung nimmt TNFα bei der Endothelzellaktivierung während des Entzündungsprozesses ein. Hierbei stellt die Aktivierung der vaskulären Endothelzellen einen wesentlichen Schritt in der Initiationsphase der entzündlichen Reaktionen im Gewebe dar. So führen pro-inflammatorische Zytokine, mit TNFα an der Spitze, zur Expression endothelialer Adhäsionsmoleküle und chemotaktisch wirksamer Chemokine, die ihrerseits Makrophagen und T-Lymphozyten die Möglichkeit geben, am Endothel anzudocken und über eine aktive Wanderung ins entzündliche Gewebe (Extravasion) zu kommen. Man unterscheidet heute in diesem Zusammenhang eine lokale Wirkung von TNFα von einer systemischen. Die lokalen Effekte sind wie oben angeführt eine verstärkte Diapetese von Immun- und Entzündungszellen ins entzündliche Gewebe und eine starke Adhäsion von Thrombozyten an den Blutgefäßwänden. Der systemische Effekt von TNFα führt zu Ödemen, einer Verringerung des Blutvolumens, Hypoproteinämie, verbreitete intravaskuläre Blutgerinnung und in ihrer Maximalvariante zu multiplem Organversagen (septischer Schock).

TNFα bewirkt also eine lokale Aktivierung des vaskulären Endothels, eine Freisetzung von Stickoxid (NO) mit nachfolgender Steigerung der vaskulären Permeabilität, eine erhöhte Expression von Adhäsionsmolekülen und eine erhöhte Expression von "class II major histocompatibility molecules" (MHC II). Das Ergebnis ist ein Einwandern von Entzündungs- und Immunzellen, Antikörpern und Komplementfaktoren in das entzündliche Gewebe. TNFα verursacht gleichfalls in den lokalen Lymphknoten eine antigenspezifische Aktivierung der B- und T-Lymphozyten. Des Weiteren aktiviert TNFα Thrombozyten und verstärkt deren Adhäsion an den Gefäßwänden.

TNFα selbst induziert die Synthese anderer pro-inflammatorischer Zytokine wie IL-1, IL-6, IL-8 und GM-CSF und führt dadurch zu einem Circulus vitiosus des entzündlichen Prozesses. Zusätzlich ist TNFα noch maßgeblich in weitere pathophysiologische Prozesse, wie die Gelenkknorpelzerstörung bei rheumatischen Erkrankungen, Knochenresorptionsprozesse, Hemmung der Knochenbildung, Hemmung der Proteoglycansynthese und Induktion von Matrix Metalloproteinasen (MMP's) und Prostaglandin E₂ *(Mease P (2002) Psoriatic arthritis: The role of TNF inhibition and the effect of its inhibition with etanercept. Clin Exp Rheumatol 20 (Suppl. 28) S116-S121)* involviert.

**Übersicht der abgesicherten biologischen Wirkung von TNFα auf humane Zellen/Organe**

| **Zellart/Organe** | **Biologische Wirkung** |
|---|---|
| | |
| Endothelzelle | Zytokin-Freisetzung, Expression von Adhäsionsmolekülen, Freisetzung von Gerinnungsfaktoren, Induktion der induzierbaren Stickoxid Synthase (iNOS) |
| B-Lymphozyt | Verstärkung der Antikörperproduktion |
| T-Lymphozyt | T-Zell Aktivierung, Apoptose, Freisetzung von IFN_{γ}, IL-2 |
| Gehirn | Induktion von Fieber und Schlaf |
| Leber | Freisetzung von "Akute Phase" Proteinen |
| Adipozyt | Hemmung der Lipoprotein-Lipase, Vermittlung der Insulin Resistenz |
| Fibroblast | Zellteilung, Induktion von MMP's und Zytokinen |
| Myozyt | Induktion der Apoptose, Funktionsstörungen |
| Osteoclast | Knochenresorption |
| Macrophage | Freisetzung von TNFα nach Stimulation durch Bakterien, Viren und Zytokine |
| Monozyt | Monozyten Aktivierung, Freisetzung von TNFα, IL-1, IL-6, iNOS |
| Thrombozyten | verstärkte Thrombozytenaggregation |

### Entwicklung von TNFα Inhibitoren

In der Vergangenheit gab es zahlreiche therapeutische Strategien, um die biologische Aktivität von TNFα zu hemmen und damit den chronischen Entzündungsprozess zu unterbrechen.
- An erster Stelle standen Bemühungen, die Synthese von TNFα zu hemmen. Hierzu kamen anti-inflammatorische Zytokine, wie z.B. das IL-10, Pentoxifylline, Thalidomid bzw.-Analoga, Kortikosteroide, Cyclosporin A, PDE-4 Inhibitoren und Antisense Oligonukleotide zum Einsatz.
   Am erfolgreichsten werden seit Jahren Kortikosteroide in der akuten Phase eines schweren entzündlichen Prozesses eingesetzt. Ihre breitere und besonders längere Anwendung ist auf Grund der schweren unerwünschten Arzneimittelwirkungen stark limitiert. Diese Gründe treffen auch für das gleichfalls seit Jahren zugelassene Immunsuppresivum Cyclosporin A zu. Pentoxifylline und Thalidomidanaloga zeigten in den klinischen Studien nur eine unzureichende therapeutische Wirksamkeit.
   Der Einsatz von PDE-4 Inhibitoren zeigt über die intrazelluläre Steigerung der cAMP Konzentration einen inhibierenden Einfluss auf die TNFα Freisetzung. Momentan sind mit Cilomilast, AWD 12-81 (GSK) und Roflumilast (Altana) drei Entwicklungskandidaten in der fortgeschrittenen klinischen Prüfung bzw. stehen kurz vor der Zulassung. Die klinische Anwendung dieser Substanzen geht jedoch auch mit unerwünschten Arzneimittelwirkungen, hauptsächlich emetischer Natur, einher. Die Antisense-Therapie befindet sich noch in einer sehr frühen Entwicklungsphase und hat zumindest in den ersten Tieruntersuchungen noch nicht die erhoffte Wirksamkeit nachweisen können.
- Ein weiterer Ansatz bestand in der Inhibierung des TNFα Prozessings durch Inhibitoren der Metalloproteinase TNF converting enzyme (TACE).
   Die Entwicklung von niedermolekularen TACE Inhibitoren befindet sich noch in der Phase der angewandten Grundlagenforschung. So beschrieben TSUKIDA et al. 2004 Hydroxamsäurederivate, die TACE in vitro hemmen *(Tsukida T, Moriyama H, Inoue Y, Kondo H, Yoshino K, Nishimura S (2004) Synthesis and biological activity of selective azasugar-based TACE inhibitors. Bioorg Med Chem Lett. 22 1569-1572).* Einige Matrix Metalloproteinase Inhibitoren hemmen auch unspezifisch TACE, sind aber auf Grund ihrer MMPinhibitorischen Aktivität für eine pharmazeutische Entwicklung nicht geeignet. WILLIAMS et al. konnten überraschend zeigen, dass der MMP Inhibitor BB-2275 paradoxerweise sowohl TACE, als auch das Shedding der TNFα Rezeptoren (TNFR1, TNFR2) hemmt und dadurch keinen TNFα inhibierenden Effekt hatte *(Williams LM, Gibbons DL, Gearing A, et al. (1996) Paradoxical effects of a synthetic metalloproteinase inhibitor that blocks both p55 and p75 TNF receptor shedding and TNF alpha processing in RA synovial membrane cell cultures. J Clin Invest 97 2833-2841).* Alle heute bekannten TACE Inhibitoren sind in ihrer hemmenden Wirkung unspezifisch, d.h. auch andere wichtige Metalloenzyme werden durch sie gehemmt, mit der Wahrscheinlichkeit unerwünschter (Neben)Wirkungen.
- In neuerer Zeit haben sich Strategien zur Blockierung des TNFα durch Antikörper gegen TNFα und lösliche TNF-Rezeptoren durchgesetzt.
   Zum jetzigen Zeitpunkt sind mit Remicade® und Humira™ zwei monoklonale anti-TNF Antikörper von der FDA und auch von der EMEA als anti-inflammatorische Therapeutika zugelassen worden.
   Remicade (Essex/Centrocor) wurde durch die FDA 1998 für die Indikation Mb. Crohn und in 2000 für die Indikation Rheumatoide Arthritis zugelassen. Momentan laufen klinische Studien für die Anwendung bei Psoriasis vulgaris und Psoriasis arthropatica. Bei Remicade handelt es sich um einen chimaeren monoklonalen Antikörper gegen das humane TNFα. In den klinischen Studien zeigte das Präparat gute bis sehr gute Wirkeigenschaften beim Mb. Crohn. Jedoch wurde über teilweise erhebliche Nebenwirkungen, wie erhöhte Infektionsgefahr, Magen-Darm Beschwerden, Kopfschmerz und allergische Reaktionen berichtet. Ein Teil der Nebenwirkungen wird auf den Maus-Anteil des monoklonalen Antikörpers zurückgeführt, der vom menschlichen Organismus als "fremd" erkannt wird, wodurch Antikörper dagegen gebildet werden. Remicade wird intravenös verabreicht und die jährlichen Medikamentenkosten belaufen sich auf über $ 12.000 pro Patient.
   Humira (Abbott) ist für die Behandlung der Rheumatoiden Arthritis seit 2002 in USA und seit 2003 in Europa zugelassen. Klinische Studien zur Behandlung der Psoriasis vulgaris zeigten sehr gute Therapieerfolge. Als häufige Nebenwirkungen wurden Kopfschmerz, erhöhte Infektanfälligkeit, Magen-Darm Beschwerden und allergische Reaktionen beobachtet. Bei Humira handelt es sich um einen vollhumanisierten monoklonalen Antikörper gegen humanes TNFα. Das Präparat wird subcutan (s.c.) verabreicht. Die jährlichen Behandlungskosten belaufen sich auch bei diesem Präparat auf über $ 12.000 pro Patient.
   Enbrel (Immunex/Wyeth) wurde erstmals durch die FDA 1998 für die Indikation Rheumatoide Arthritis zugelassen und seit 2000 ist das Präparat auch auf dem europäischen Markt. Die Zulassung für die Indikation Psoriasis vulgaris und Psoriasis arthropatica wird noch 2004 von der FDA erwartet. Bei Enbrel handelt es sich um ein rekombinantes (CHO-Zellen) dimeres Fusionsprotein, bei dem zwei extrazelluläre Bindungsdomänen des p75-Anteils des TNF Rezeptors an den Fc-Anteil des humanen IgG1-Moleküls angekoppelt sind und dadurch lösliches TNFα im Blut/Gewebe abbinden und somit neutralisieren kann. Laut Herstellerangaben besitzt dieses Fusionsprotein ein geringes immunogenes Potential, wohingegen aber auch Fälle beschrieben wurden, in denen eine Antikörperbildung gegen das Fusionsprotein beobachtet wurde. Als häufige Nebenwirkungen wurden allergische Reaktionen, Infektanfälligkeit und die Bildung von Auto-Antikörpern (ANA) beschrieben. Das Präparat wird subcutan verabreicht und die jährlichen Behandlungskosten belaufen sich gleichfalls auf über $ 10.000 pro Patient.

Zusammenfassend kann festgestellt werden, dass sich das therapeutische Konzept der Hemmung von TNFα als biologischer Endpunkt, als tragfähig in der Klinik erwiesen hat. Momentan stehen nur proteinogene Präparate (monoklonale Antikörper, Fusionsproteine) zur Verfügung, die eine Reihe von unerwünschten Arzneimittelwirkungen aufweisen. Darüber hinaus ist deren intravenöse bzw. subkutane Applikationsform für Patienten sehr belastend und geht mit einer entsprechend schlechten Compliance einher. Die sehr hohen Herstellungs- und somit auch Behandlungskosten limitieren ebenfalls deren Einsatz.

Es besteht deshalb der dringende Bedarf, neuartige nebenwirkungsarme PDE-4 Inhibitoren mit besserer therapeutischer Breite zu entwickeln und in die Klinik einzuführen. Es besteht deshalb außerdem nach wie vor das dringende Bedürfnis nach niedermolekularen, nichtproteinogenen Hemmern der Freisetzung von TNF-alpha, die oral verabreicht werden können und eine bessere Verträglichkeit aufweisen. Zurzeit existieren noch keine bekannten niedermolekularen Verbindungen, die selektiv die Synthese von TNFα hemmen, ohne mit anderen Stoffwechselwegen zu interagieren.

Die Aufgabe der vorliegenden Erfindung ist daher neue Verbindungen bereitzustellen, die selektiv das Enzym PDE-4 und/oder die Freisetzung von TNF-alpha hemmen.

Gelöst wird diese Aufgabe durch die nachfolgend gezeigten Verbindungen aus der Klasse der anellierten Pyrido[3',2':4,5]thieno[3,2-d]pyrimidin- und anellierten Pyrido[3',2':4,5]furo[3,2-d]pyrimidin-Derivate der Formel 1, die vorrangig PDE-4 Inhibitoren darstellen, aber auch eine Inhibierungswirkung auf die Freisetzung von TNF-apha zeigen. Die Aufgabe wird weiter durch die Pyrido[3',2':4,5]thieno[3,2-d]pyrimidin-2,4-dione und -4-one sowie Pyrido[3',2':4,5]furo[3,2-d]pyrimidin-2,4-dione und -4-one der allgemeinen Formeln 4a und 4b gelöst, die zu einer signifikanten Inhibition der TNFα-Freisetzung führen.

Über biologisch aktive Pyrido[3',2':4,5]thieno[3,2-d]pyrimidin-Derivate, welche am Pyridin-Teil [c]-anelliert sind, ist bisher nur wenig berichtet worden.

So wurden von *S. Leistner et al.: Ger. (East) (1988), DD 258013,* die Synthese von 8,9,10,11-Tetrahydropyrimido[4',5':4,5]thieno[2,3-c]isochinolin-4-onen beschrieben, welche antianaphylaktische und antiinflammatorische Eigenschaften besitzen. Die Synthese von 1,2-Dihydro-pyrano[4',3':4,5]pyrido[2,3-b]thieno[3,2-d]pyrimidin-8-onen, welche antibakterielle Eigenschaften zeigen, wurde *von Paronikyan et al.: U.S.S.R. (1988), SU 85-3914497* beschrieben. Desweiteren wurden am Pyridin-Teil [c]-anellierte Pyrido[3',2':4,5]thieno[3,2-d]pyridin-2-carbonsäuren von *H. Vieweg et al.: Ger. (East) (1988), DD 258018,* und von S. *Leistner et al.: Ger. (East) (1988), DD 258015, DD 258017 u. DD 258019,* hinsichtlich ihrer potentiellen Anwendbarkeit als Arzneistoffe erwähnt.

Als Inhibitoren der PDE 4 sind anellierte Pyrido[3',2':4,5]thieno[3,2-d]pyrimidin- und anellierte Pyrido[3',2':4,5]furo[3,2-d]pyrimidin-Derivate bisher jedoch völlig unbekannt.

Als Inhibitoren der TNFα-Freisetzung sind Pyrido[3',2':4,5]thieno[3,2-d]pyrimidin-2,4-dione und -4-one sowie Pyrido[3',2':4,5]furo[3,2-d]pyrimidin-2,4-dione und -4-one der allgemeinen Formeln 4a und 4b auch bisher völlig unbekannt.

### Detailierte Beschreibung der Erfindung

Die Erfindung betrifft substituierte anellierte Pyrido[3',2':4,5]thieno[3,2-d]pyrimidine und anellierte Pyrido[3',2':4,5]furo[3,2-d]pyrimidine der allgemeinen Formel 1 sowie substituierte anellierte Pyrido[3',2':4,5]thieno[3,2-d]pyrimidin-2,4-dione bzw. -4-one und substituierte anellierte Pyrido[3',2':4,5]furo[3,2-d]pyrimidin-2,4-dione bzw. -4-one der allgemeinen Formel 4a bzw. 4b. 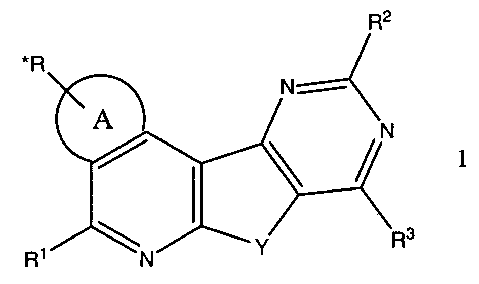 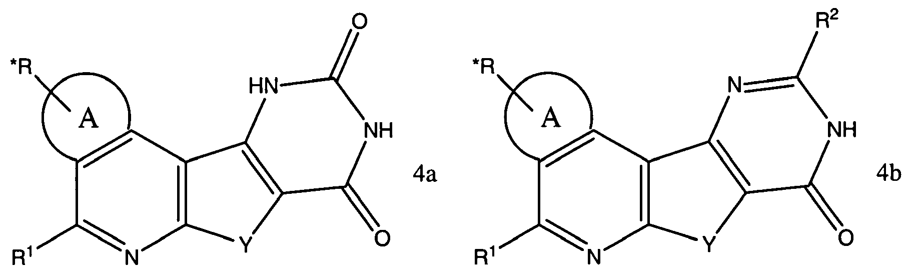
worin bedeuten:
- Y: - Schwefel oder Sauerstoff,
- A: - anellierter Carbocyclus mit 5 bis 8 Ringatomen, einfach oder mehrfach ungesättigt sowie aromatisch, (ggf. mit R^{*} substituiert und>/oder ggf. mit R^{§} substituiert);
- anellierter Bicyclus mit 6 bis 11 Ringatomen, einfach oder mehrfach ungesättigt, welcher auch Stickstoff, Sauerstoff und/oder Schwefel enthalten kann (ggf. mit R* substituiert und/oder ggf. mit R^{§} substituiert);
- annelierter Heterocyclus mit 5 bis 8 Ringatomen einfach oder mehrfach ungesättigt sowie aromatisch, welcher Stickstoff, Sauerstoff und Schwefel einfach, mehrfach, gleich oder ungleich unabhängig voneinander im Cyclus enthalten kann (ggf. mit R* substituiert und/oder ggf. mit R^{§} substituiert),
- R¹: - Wasserstoff (außer wenn A ein anellierter unsubstituierter Cyclopenten-Ring kombiniert mit R²,R³ gleich -OH ist)
- C₁₋₁₀Alkyl (ggf. mit R^{§} substituiert),
- C₂₋₁₂ Alkenyl und C₂₋₁₂ Alkinyl (jeweils ggf. mit R^{§} substituiert ),
- Difluormethyl, Trifluormethyl,
- Benzyl, Phenyl-(C₂₋₆)alkyl (jeweils ggf. mit R^{§} substituiert), Phenyl (ggf. mit R^{§} substituiert), dabei inbesondere: 4-R^{§} -Phenyl, 3-R^{§} -Phenyl, 2-R^{§}-Phenyl, 3-R^{§},4-R^{§}-Phenyl, 3-R^{§},4-R^{§}-,5-R^{§}-Phenyl, 2-R^{§}, 4-R^{§}-Phenyl,
- 1-Naphthyl, 2-Naphthyl (jeweils ggf. mit R^{§} substituiert),
- C₃₋₁₄Cycloalkyl, C₃₋₁₄Cycloalkenyl (jeweils ggf. mit R^{§}-substituiert),
- mono- oder bicyclische gesättigte oder ein- oder mehrfach ungesättigte Heterocyclen mit 5 - 14 Ringatomen darunter 1 - 4 Heteroatome, die vorzugsweise N, O und S sind,
- C₂₋₁₂Alkylacyl (ggf. mit R^{§} substituiert),
- Benzoyl, 1- und 2-Naphthoyl (jeweils ggf. mit R^{§} substituiert),
- Heterocyclylacyl [z.B. Nicotinoyl, Isonicotinoyl, 2-Picolinoyl, 2-Thienoyl, 2-Furoyl] (ggf. mit R^{§} substituiert)
- Hydroxy,
- Sulfhydryl,
- C₁₋₁₀ Alkoxy,
- Alkylthio, Alkylsulfinyl und Alkylsulfonyl (jeweils C₁₋₆)
- Formyl, Carboxyl, C₁₋₄Alkoxycarbonyl;
- CONH₂, CONHAlk und CONAlk₂ (mit "Alk" jeweils C₁₋₆),
- Cyano, Rhodano, Nitro, SO₃H SO₂OAlk (mit "Alk": C₁₋₅),
- Chlor, Brom, Iod, Fluor,
- Amino, C₁₋₆Alkylamino, Di(C₁₋₅)alkylamino (jeweils ggf. mit R^{§} am Alkylrest substituiert),
- Morpholino, Thiomorpholino, Thiomorpholino-S-Oxid, Thiomorpholino-S,S-Dioxid, Pyrrolidino,
- Piperidino, 1-Piperazino, 4-Methyl-1-piperazino, 4-Hydroxyethyl-1-piperazino, 4-Phenyl-1-piperazino,
- Cycloalkylamino, C₃₋₁₄ Arylamino und Heteroarylamino [z.B. Phenyl-, 1- und 2-Naphthyl-, 2-, 3- oder 4-Pyridyl-, Chinolinyl-, Isochinolinyl-, Acridinyl-, Phenothiazinyl-, 2-Thienyl- und 2-Furylamino] (ggf. jeweils an den carbo- bzw. heterocyclischen Ringen mit R^{§} substituiert);
- R²: - C₂₋₁₄Alkyl, C₃₋₁₄Cycloalkyl, C₂₋₁₄Alkenyl, C₃₋₁₄Cycloalkenyl, C₂₋₁₄Alkinyl (jeweils ggf. am C-Skelett der vorgenannten Reste mit R^{§} substituiert);
- Benzyl, Phenyl-(C₂₋₆)alkyl (jeweils ggf. mit R^{§} substituiert), Phenyl (ggf. mit R^{§} substituiert), dabei inbesondere: 4-R^{§} -Phenyl, 3-R^{§} -Phenyl, 2-R^{§}-Phenyl, 3-R^{§},4-R^{§}-Phenyl, 3-R^{§},4-R^{§}-,5-R^{§}-Phenyl, 2-R^{§}, 4-R^{§}-Phenyl,
- 1-Naphthyl, 2-Naphthyl (jeweils ggf. mit R^{§} substituiert);
- S-Alkyl (C₃-C₈), SO-Alkyl, SO₂-Alkyl, (jeweils C₁-C₈), S-Alkenyl, SO-Alkenyl, SO₂-Alkenyl (jeweils C₂-C₈) S-Alkinyl, SO-Alkinyl, SO₂-Alkinyl (jeweils C₂-C₆) (ggf. jeweils am C-Skelett der vorgenannten Reste mit -OH, -CN, - SCN, -NO₂, Phenyl oder C₃ - C₇Cycloalkyl substituiert);
- mono-, bi- oder tricyclische gesättigter oder ein- oder mehrfach ungesättigter heterocyclischer Rest mit insgesamt 4-14 Ring-Atomen, davon 1-5 Heteroatomen, die vorzugsweise N, O, S und Se sind (jeweils ggf. mit R^{§} substituiert);
- OR⁴, worin R⁴ bedeutet
   - Wasserstoff
   - CH₃, C₂H₅, (CH₂)₂CH₃, (CH₂)₃CH₃, CH(CH₃)₂, CH₂CH(CH₃)₂, C(CH₃)₃, CH₂CH₂OH, CH₂CH₂SH, CH₂CH₂SCH₃, CH₂CF₃, CH₂CCl₃, CH₂CHF₂, CH₂CHCl₂, CH₂CH₂F, CH₂CH₂Cl, CH₂CH₂Br,
   - C₃₋₇Cycloalkyl [z.B.Cylopropyl, Cylopropylmethyl, Cylobutyl, Cyclobutylmethyl, Cyclopentyl, Cyclopentylmethyl, Cylohexyl, Cyclohexylmethyl] (ggf. am C- Skelett mit R^{§} substituiert),
   - C₂₋₅Alkenyl und C₂₋₅Alkinyl,
   - C₃₋₇ Cycloalkenyl,
   - Aryl und Heteroaryl als Reste mono-, bi- oder tricyclischer Aromaten oder Heteroaromaten mit ggf. 6-14 Ring-Atomen [z.B. Phenyl, 4-R^{§} -Phenyl, 3-R^{§} -Phenyl, 2-R^{§}-Phenyl, 3-R^{§},4-R^{§}-Phenyl, 3-R^{§},4-R^{§}-,5-R^{§}-Phenyl],
   - 1-Naphthyl, 2-Naphthyl (ggf. jeweils mit R^{§} substituiert),
   - Pyridyl, Isochinolinyl, Chinolinyl, Acridinyl;
- NR⁵R⁶, worin dieser Substituent insgesamt bedeutet:
   - Morpholino, Thiomorpholino, Thiomorpholino-S-Oxid, Thiomorpholino-S,S-Dioxid, Pyrrolidino, Piperidino, 1-Piperazino, 1-Homopiperazino, 4-C₁₋₆-Alkyl-1-piperazino, 4-(2-Hydroxyethyl)-1-piperazino, 4-Benzyl-1-piperazino, 4-Aryl-1-piperazino (ggf. mit R^{§} am heterocycloaliphatischen Ring substituiert),
   - weitere Amino-Reste sekundärer, mono- oder polycyclischer cycloaliphatischer Amine mit insgesamt 5-14 Ringatomen, einschließlich der am C-Skelett mit R^{§} substituierte Vertreter;
   - Amino-Reste sekundärer aliphatischer und aromatischer Amine, R⁵R⁶NH,
      worin R⁵ und R⁶ unabhängig voneinander gleich oder ungleich sein können und bedeuten:
   - C₁₋₆Alkyl, Benzyl, Phenyl, 1- und 2-Naphthyl, 2-, 3- und 4-Pyridyl, Chinolinyl, Isochinollnyl, 2-Thienyl, 2-Furyl (ggf. jeweils mit R^{§} substituiert);
   - Aminoreste primärer Amine, R⁵NH₂, (worin R⁵ dasselbe wie oben bedeutet),
   - Amino, NH₂, mit der Einschränkung, dass dann R³ nur die Bedeutung von OR⁴ hat ;
- R³: - OR⁴, wobei R⁴ dasselbe wie oben bedeutet,
- NR⁵R⁶, mit gleicher Bedeutung wie oben
- Azido, Hydroxylamino, O-(C₁₋₃)Alkylhydroxylamino,
- N-(C₁₋₃)Alkylhydroxylamino, N,N-Di(C₁₋₃)alkylhydroxylamino,
- Hydrazino, (C₁₋₄)Alkyl- und Di(C₁₋₄)alkylhydrazino,
- Benzylhydrazino, Acylhydrazino, N,N-Diacylhydrazino,
- Carbamoylamino,
- 1-Imidazolyl, 1,2,4-Triazol-1yl, 1-Pyridinium,
- 1-Pyrazinium, 1-Pyridazinium, einschliesslich der alkylsubstituierten Vertreter dieser Azaheterocyclen;

Der oben erwähnte Ausdruck "ggf. mit R* substituiert" bedeutet, daß die genannten Reste einfach oder mehrfach, gleich oder ungleich unabhängig voneinander, substituiert sein können, wobei R* folgende Bedeutung hat:
=O, =S, =N-H, =N-C₁₋₈Alkyl, =N-Aryl, =N-OH, =N-O-C₁₋₈Alkyl, =N-O-C₁₋₁₄Aryl

Der oben erwähnte Ausdruck "ggf. mit R^{§} substituiert" bedeutet, daß die genannten Reste einfach oder mehrfach, gleich oder ungleich unabhängig voneinander, substituiert sein können, wobei R^{§} folgende Bedeutung hat:
- OH, -SH, -O-C₁₋₈Alkyl, -O-C₆₋₁₄ Aryl, -S-C₁₋₄ Alkyl, -S-C₆₋₁₄Aryl,
- SO-C₁₋₄Alkyl, -SO-C₆₋₁₄Aryl, -SO₂-C₁₋₄Alkyl, -SO₂-C₆₋₁₄Aryl, -SO₃H,
- OSO₂C₁₋₈Alkyl, -OSO₂C₆₋₁₄Aryl, -COOH, -COOC₁₋₈Alkyl, -(CO)C₁₋₈Alkyl,
- COOH, -CONH₂, -CONHC₁₋₆Alkyl, -CON(C₁₋₆Alkyl)₂,
- NH₂, -NHC₁₋₆Alkyl, -N(C₁₋₆Alkyl)₂, -NHC₆₋₁₄Aryl, -NH-Hetaryl,
- N(C₆₋₁₄Aryl)₂, -N(C₁₋₆Alkyl)(C₆₋₁₄Aryl),
- CH₃, -CHF₂, -CF₃, -C₂H₅, -C(CH₃)₂, -(CH₂) ₂CH₃, -(CH₂) ₃CH₃, -CH₂CH₂OH,
- CH₂CH₂SH, -CH₂CH₂SCH₃, -CH₂CF₃, -CH₂CCl₃, -CH₂CHF₂, -CH₂CHCl₂,
- CH₂CH₂F, -CH₂CH₂Cl, -CH₂CH₂Br, -Cylopropyl, -Cylopropylmethyl,
- Cylobutyl, -Cyclobutylmethyl, -Cyclopentyl, -Cyclopentylmethyl,
- Cylohexyl, -Cyclohexylmethyl,
- F, -Cl, -Br, -I, -CN, -NO₂, und -SCN.

Die Begriffe "Alkyl, Alkenyl, Alkinyl, Alkoxy, usw.", auch in Wortzusammensetzungen wie Alkylsulfonyl, Alkylamino oder Alkoxycarbonyl usw. bedeuten sowohl die unverzweigten wie auch die verzweigten möglichen Verbindungen. Ebenso bedeuten "Alkenyl und Alkinyl" die entsprechend möglichen einfach oder mehrfach ungesättigten Verbindungen. Das gleiche gilt auch für die entsprechenden cyclischen Verbindungen.

Ausgeschlossen werden folgende Verbindungen:
1,4-Dihydro-2,2-dimethyl-5-(4-morpholinyl)-10-propylthio-2H-pyrano[4",3":4',5']pyrido[3',2':4',5']thieno[3,2-d]pyrimidin-8(9H)-on,
10-Buthylthio-1,4-dihydro-2,2-dimethyl-5-(4-morpholinyl)-2H-pyrano[4",3":4',5']pyrido[3',2':4',5']thieno[3,2-d]pyrimidin-8(9H)-on

und 2-Methyl-5-thiophen-2-yl-1,2,3,4-tetrahydro-11H-7-thia-2,6,9,11-tetraaza-benzo[c]fluorene-8,10-dione

Von den Verbindungen der allgemeinen Formel 1 sind als besondere Ausführungsform der Erfindung die Verbindungen der allgemeinen Formeln 1 a und 1 b hervorzuheben, 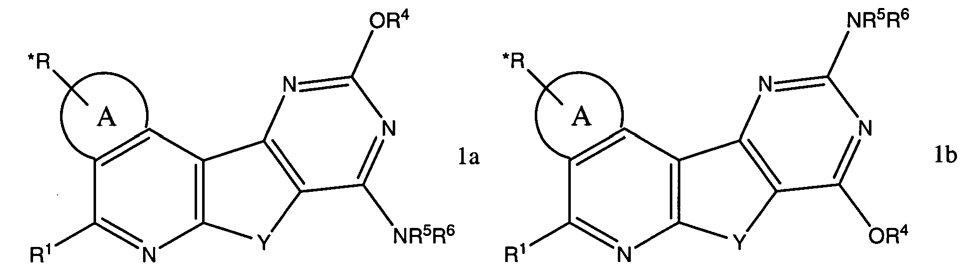
worin R¹, R⁴, R⁵, R⁶ sowie A, R* und Y die oben genannten Bedeutungen besitzen.

Die Erfindung betrifft auch physiologisch verträgliche Salze der Verbindungen der allgemeinen Formeln 1, 1 a und 1 b sowie 4a und 4b.

Die physiologisch verträglichen Salze werden auf übliche Weise durch Umsetzung basischer Verbindungen der allgemeinen Formeln 1, 1 a und 1 b sowie 4a und 4b mit anorganischen oder organischen Säuren, ggf. auch bei Vorliegen von Verbindungen mit aciden Eigenschaften, wenn z.B. einer der Substituenten R¹, R² oder R³ in diesen Verbindungen -COOH bzw. -SO₃H bedeutet, durch Neutralisation mit anorganischen oder organischen Basen, erhalten.

Als anorganische Säuren kommen vorzugsweise Salzsäure, Schwefelsäure, Salpetersäure oder Bromwasserstoffsäure, als organische Säuren zum Beispiel Ameisensäure, Essigsäure, Propionsäure, Glykolsäure, Milchsäure, Mandelsäure, Weinsäure, Äpfelsäure, Zitronensäure, Malonsäure, Maleinsäure, Fumarsäure, Succinsäure, Alginsäure, Benzoesäure, 2-, 3- und 4-Alkyloxy- und Acyloxybenzoesäuren, Ascorbinsäure, C₁-C₃,Alkylsulfonsäuren, Benzolsulfonsäure, Nicotinsäure, Isonicotinsäure und Aminosäuren zur Anwendung.

Als anorganische Basen kommen zum Beispiel Ammoniak, Natron- und Kalilauge sowie als organische Basen Alkylamine, C₁-C₃, Pyridin, Chinolin, Isochinolin, Piperazin und -Derivate, Picoline, Chinaldin oder Pyrimidin zur Anwendung.

Weiterhin können physiologisch verträgliche Salze der Verbindungen gemäß der allgemeinen Formeln 1, 1 a und 1 b sowie 4a und 4b dadurch gewonnen werden, dass jene Substanzen, die als Substituenten eine tertiäre Amino-Gruppe besitzen, in prinzipiell bekannter Weise mit alkylierenden Agentien - wie zum Beispiel Alkyl- oder Aralkylhalogeniden - in die entsprechenden quarternären Ammoniumsalze übergeführt werden können.

Die Erfindung betrifft auch Solvate der Verbindungen, einschließlich der pharmazeutisch akzeptablen Salze, Säuren, Basen und Ester sowie deren aktive Metabolite und gegebenenfalls deren Tautomere gemäß der allgemeinen Formeln 1, 1 a und 1b sowie 4a und 4b einschließlich Prodrug-Formulierungen. Prodrug-Formulierungen umfassen hierbei alle jene Substanzen, die durch einfache Transformation einschließlich Hydrolyse, Oxidation, oder Reduktion entweder enzymatisch, metabolisch oder auf andere Art und Weise entstehen. Insbesondere, wenn ein solches Prodrug dieser erfindungsgemäßen Verbindungen einem Patienten appliziert wurde, und dieses Prodrug in eine Substanz der allgemeinen Formel 1, 1 a und 1b sowie 4a und 4b transformiert wird, wodurch der gewünschte pharmakologische Effekt erzielt wird.

Die durch die erfindungsgemäßen Verbindungen behandelbaren Erkrankungen schließen alle ein, bei denen das Enzym Phosphodiesterase (PDE4) eine Rolle spielt, z.B. Asthma bronchiale, COPD, Rheumatoide Arthritis (RA), Osteoarthritis, Multiple Sklerose, Guillain-Barre Syndrom, Mb. Crohn, Colitis ulcerosa, Psoriasis, atopische Dermatitis, allergische Ekzeme, Rhinitis allergica, allergische Konjunktivitis, systemische Sklerodermie, Graft versus host disease (GvHD), Systemischer Lupus Erythematodes (SLE), Diabetes mellitus Typ I, Mb. Alzheimer, posttraumatisches Multiorganversagen, Toxisches Schocksyndrom, Akute Glomerulonephritis, akute und chronische Schmerzen, Arteriosklerose, Herzinfarkt, Schlaganfall, Tumorerkrankungen und hier insbesondere Tumoren des blutbildenden Systems wie z.B. Leukämien und Lymphome, virale Erkrankungen und hier insbesondere retrovirale Erkrankungen wie z.B. das erworbene Immundefizienz Syndrom (AIDS) und Myastenia Gravis.

Die durch die erfindungsgemäßen Verbindungen behandelbaren Erkrankungen schließen auch die in der Veterinärmedizin auftretenden mit ein, insbesondere das Asthma bronchiale, die COPD und Dermatiditen unterschiedlicher Genese.

Die durch die erfindungsgemäßen Verbindungen behandelbaren Erkrankungen schließen darüber hinaus alle ein, bei denen TNF-alpha eine Rolle spielt und die durch eine Hemmung oder Inhibierung desselben positiv zu beeinflussen sind, z.B. chronische Entzündungserkrankungen, Autoimmun-Erkrankungen, cardiovaskuläre Erkrankungen, virale Erkrankungen und hier insbesondere retrovirale Erkrankungen wie z.B. das erworbene Immundefizienz Syndrom (AIDS) sowie Krebs, insbesondere Entartungen des blutbildenden Systems. Insbesondere sind dies Rheumatoide Arthritis, Osteoarthritis, Osteoporosis, Asthma bronchiale, chronische obstruktive pulmonäre Erkrankung (COPD), Multiple Sklerose, Sepsis, cerebrale Form der Malaria, neurodegenerativen Erkrankungen wie z.B. Mb. Alzheimer, Mb. Parkinson, Guillain-Barre-Syndrom, Crohns Disease, Colitis ulcerosa, Psoriasis, Graft-versus-Host-Disease (GvHD), systemischer Lupus erythomatodes (SLE), Vasculitis, Uveitis, insulin-abhängiger Diabetes mellitus, Respiratorisches Distress-Syndrom beim Erwachsenen (ARDS), multiples Organversagen nach Trauma, aktute Glomerulonephritis, akute und chronische Schmerzen, Arteriosklerose, Herzinfarkt, Schlaganfall, entzündliche Dermatosen, atopische Dermatitis, Psoriasis vulgaris, Alopecie, Rhinitis allergica, allergische Konjunktivitis, akute Meningitis, Myastenia Gravis, Sklerodermie und Sarkoidose.

Die erfindungsgemäßen Verbindungen können auf verschieden Wegen verabreicht werden, z.B. oral, parenteral, kutan, subkutan, intravenös, intramuskulär, rektal oder inhalativ. Bevorzugt ist die orale oder inhalative Verabreichung. Die Verbindung wird einem Patienten, der eine Therapie einer unter das Indikationsspektrum der erfindungsgemäßen Verbindungen fallenden Krankheit bedarf, über einen vom Arzt zu bestimmenden Zeitraum verabreicht. Die Verbindung kann sowohl Menschen als auch anderen Säugern verabreicht werden.

Die Dosierung der erfindungsgemäßen Verbindungen wird vom Arzt anhand der patientenspezifischen Parameter wie z.B. Alter, Gewicht, Geschlecht, Schwere der Erkrankung, etc. bestimmt. Bevorzugt beträgt die Dosierung zwischen 0,001 mg/kg bis 100.000 mg/kg Körpergewicht, bevorzugt 0,01 bis 10.000 mg/kg Körpergewicht und ganz bevorzugt 0,1 bis 1.000 mg/kg Körpergewicht.

Entsprechend der Art der Verabreichung wird das Medikament in geeigneter Weise formuliert, z.B. in Form von Lösungen bzw. Suspensionen, einfachen oder dragierten Tabletten, Hart- oder Weichgelatinekapseln, Pulver zur Rekonstitution vor Gebrauch, Aerosolen, Inhalationssprays, Wirkstoffpflastern, Granulaten, Suppositorien, Ovula, Injektionspräparaten, Cremes, Salben, Gels, Mikrospheren, Implantaten, die nach üblichen galenischen Verfahren hergestellt werden.

Die erfindungsgemäßen Verbindungen können gegebenenfalls zusammen mit weiteren Wirkstoffen und mit in pharmazeutischen Zusammensetzungen üblichen Exzipientien formuliert werden, z.B. je nach herzustellendem Präparat Talk, Gummi arabicum, Lactose, Stärke, Magnesiumstearat, Kakaobutter, wäßrige und nichtwäßrige Träger, Fettkörper mit tierischem oder pflanzlichem Ursprung, Paraffinderivate, Glykole (insbesondere Polytethylenglykol), verschiedene Weichmacher, Dispergiermittel oder Emulgatoren, pharmazeutisch verträgliche Gase (z.B. Luft, Sauerstoff, Kohlendioxid usw.), Konservierungsstoffe.

Zur Herstellung flüssiger Präparate können Additive wie Natriumchloridlösung, Ethanol, Sorbit, Glycerin, Olivenöl, Mandelöl, Propylenglycol, Ethylenglycol oder andere Additive, die in der Pharmazie üblichen sind, verwendet werden.

Bei der Verwendung von Infusions- oder Injektionslösungen sind diese bevorzugt wäßrige Lösungen oder Suspensionen, wobei es möglich ist, diese vor Gebrauch herzustellen, beispielsweise aus lyophilisierten Präparaten, die den Wirkstoff alleine oder zusammen mit einem Träger, wie Mannit, Lactose, Glucose, Albumin und dergleichen, enthalten. Die gebrauchsfertigen Lösungen werden sterilisiert und gegebenenfalls mit Hilfsmitteln vermischt, beispielsweise mit Konservierungsstoffen, Stabilisatoren, Emulgatoren, Lösungsvermittlern, Puffern und/oder Salzen zur Regulierung des osmotischen Drucks. Die Sterilisierung kann durch Sterilfiltration durch Filter mit einer kleinen Porengröße erzielt werden, wonach die Zusammensetzung gegebenenfalls lyophilisiert werden kann. Geringe Mengen an Antibiotika können auch zugesetzt werden, um die Beibehaltung der Sterilität zu gewährleisten.

Weiter bevorzugt werden Inhalationszusammensetzungen, z.B. in Form von Aerosolen, Sprays, oder als mikronisiertes Pulver hergestellt. Dazu werden die erfindungsgemäßen Verbindungen entweder als in pharmazeutisch üblichen Lösungsmitteln gelöst bzw. suspendiert und mittels Überdruck in einem bestimmten Volumen fein verteilt und inhaliert. Ein entsprechendes Vorgehen erfolgt bei den zu inhalierenden Festsubstanzen, die gleichfalls mittels Überdruck fein verteilt und inhaliert werden. Ebenfalls andere als mit Überdruck funktionierende Applikatoren sind hierbei eingeschlossen.

Die Erfindung betrifft auch pharmazeutische Zubereitungen, die eine therapeutisch wirksame Menge des aktiven Inhaltsstoffs (erfindungsgemäße Verbindung der Formeln (1), (4a), (4b)) zusammen mit organischen oder anorganischen festen oder flüssigen pharmazeutisch verträglichen Trägern, die für die beabsichtigte Verabreichung geeignet sind, und die mit den aktiven Inhaltsstoffen nicht nachteilig Wechselwirken, enthalten.

Die Erfindung betrifft auch Verfahren zur Herstellung pharmazeutischer Zubereitungen, die dadurch gekennzeichnet sind, dass die erfindungsgemäße Verbindung mit einem pharmazeutisch verträglichen Träger vermischt wird.

Die erfindungsgemäßen Verbindungen eignen sich auch im Rahmen von Kombinationstherapien mit schon bekannten Wirkstoffen zur Behandlung der oben genannten Erkrankungen. Dabei sollen überraschende Synergieeffekte zur Steigerung der therapeutischen Wirksamkeit der erfindungsgemäßen Substanzen genutzt werden. Die Kombination kann zum einen darin bestehen, eine einzige pharmazeutische Zusammensetzung anzubieten, die mindestens eine der erfindungsgemäßen Verbindungen in Kombination mit einem oder mehrere der nachfolgend genannten Wirkstoffen enthält oder dem Patienten werden gleichzeitig oder zeitlich versetzt mehrere Mittel, die einen oder mehreren der nachfolgenden Wirkstoffe enthalten, verabreicht.

Es ist bevorzugt eine oder mehrere der erfindungsgemäßen Verbindungen mit einem oder mehreren der folgenden Wirkstoffe zu kombinieren:
- β₂-Adrenoceptor Agonisten (z.B. Terbutalin, Salbutanol, Salmetanol, Fenoterol, Formoterol)
- Dinatriumcromoglycat
- Korticosteroide
- PDE-4 Inhibitoren (wie z.B. Roflumilast, Cilomilast, AWD 12-281)
- Leukotrien-Antagonisten (entweder Enzym-Inhibitoren [wie 5-Lipoxygenaseinhibitoren oder Arachidonsäure-Enzyminhibitoren] oder Rezeptorantagonisten) , z.B. Pramkulast, Montelukast, Zafirlukast, Zileuton
- Antihistaminika (bevorzugt solche mit Mastzellen-stabilisierenden Eigenschaften oder Leukotrien-antagonisierenden Aspekten, wie z.B. Loratadin, Astemizol, Mizolastin, Olopatadin
- Theophyllin
- Muscarinrezeptor-Antagonisten, z.B. Spiriva
- (monoklonale) Antikörper gegen TNF-alpha oder andere Wirkstoffe, die die Bildung bzw. Freisetzung von TNF-alpha oder die Aktivität von TNF-alpha hemmen (z.B. rekombinante lösliche Rezeptorkonstrukte)

Die Kombination mit Corticosteroiden, Leukotrien-Antagonisten, Antihistaminika, Theophylin, Muscarinrezeptor-Antagonisten und/oder TNF-alpha-Hemmern dient besonders dazu, den akuten zu behandelnden Krankheitszustand nicht chronisch werden zu lassen, da die erfindungsgemäßen Verbindungen und die anderen Wirkstoffe komplementäre Aspekte der der Erkrankung zugrundeliegenden pathophysiologischen Mechanismen angehen. Erfindungsgemäß sollte insbesondere die Kombination der erfindungsgemäßen Verbindungen mit β₂₋Adrenoceptoragonisten und/oder Dinatriumcromogycat als Inhalationstherapie bei milden Formen von Asthma, insbesondere im Kindesalter, wirksam sein. In der Kombination mit Glucocorticoiden ergibt sich ein positiver Effekt daraus, daß weniger Glucocorticoide angewendet werden müssen und damit ein Spareffekt zu erreichen ist und die von Glucocorticoiden bekannten Nebenwirkungen vermindert werden bzw. ganz ausbleiben. Die Kombination der erfindungsgemäßen Verbindungen mit Glucocorticoiden und/oder Theophyllin hat sich insbesondere bei persistierendem Asthma bewährt.

Abhängig von der Krankheitsausprägung und den zugrunde liegenden Symptomen können die erfindungsgemäßen Verbindungen zu den anderen Wirkstoffen in der Kombination im Verhältnis von 1:10.000 bis 10.000:1 vorliegen.

Die Erfindung betrifft weiterhin Verfahren zur Herstellung der erfindungsgemäßen Verbindungen.

Die erfindungsgemäßen Verfahren zur Herstellung der Verbindungen der allgemeinen Formeln 1, 1 a und 1b sowie 4a und 4b mit den zuvor aufgeführten Bedeutungen von R¹, R⁴, R⁵, R⁶ sowie A, R^{*} und Y sind gekennzeichnet durch folgende Verfahrensweise:
- Umsetzung der bekannten bzw. nach bekannten Methoden analog dargestellten anellierten Pyridin-3-carbonitrile der allgemeinen Formel 2 (mit identischer Bedeutung von R¹, A, R* und Y wie vorstehend)

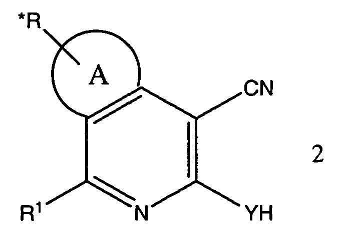

in an sich bekannter Weise mit Chloracetamid, ClCH₂CONH₂, in methanolischer oder ethanolischer Lösung in Gegenwart eines Natriumalkoxides, vorzugsweise Natriummethoxid oder Natriumethoxid, in die analogen anellierten 3-Amino-thieno[2,3-b]pyridin-2-carbonsäureamide der allgemeinen Formel 3 (mit identischer Bedeutung von R¹, A und R^{*} wie vorstehend und worin Y die Bedeutung von S aufweist), bzw. in die analogen anellierten 3-Amino-furo[2,3-b]pyridin-2-carbonsäureamide der allgemeinen Formel 3 (mit identischer Bedeutung von R¹, A und R* und wobei Y die Bedeutung von O aufweist);

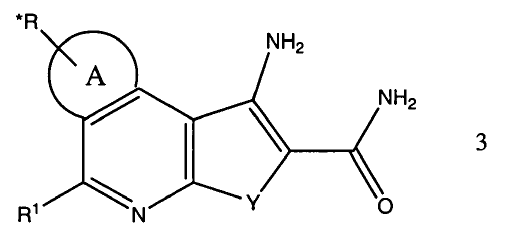

Die Verbindungen der allgemeinen Formel 3 sind aus den Verbindungen der allgemeinen Formel 2 (wobei R¹, A, R* und Y die oben genannten Bedeutungen aufweisen) auch dadurch darstellbar, dass diese Verbindungen mit Choracetamid zunächst in vorzugsweise ethanolischer Lösung in Gegenwart von vorzugsweise Triethylamin oder einem sekundären cycloaliphatischen Amin wie Morpholin, Piperidin oder Pyrrolidin umgesetzt werden und anschließend in einem weiteren Syntheseschritt in vorzugsweise wasserfreier ethanolischer Lösung mit einer katalytischen Menge Natriummethoxid oder Natriumethoxid durch Erhitzen unter Rückfluss gleichfalls in die oben genannten Verbindungen der allgemeinen Formel 3 übergeführt werden.
- Umsetzung der Verbindungen der allgemeinen Formel 3 mit Phosgen, Trichlormethylchlorformiat oder mit C₁₋₃-Alkylchlorformiaten, wie z.B. Methyl- oder Ethylchlorformiat, in einem vorzugsweise aprotischen, hochsiedenden Lösungsmittel wie Dioxan, Tetrahydrofuran oder Toluol bzw. deren Mischungen unter Erhitzen, ggf. in Anwesenheit katalytischer Mengen eines Natriumalkoxides, wie zum Beispiel Natriumethylat, zu Verbindungen der allgemeinen Formel 4a, 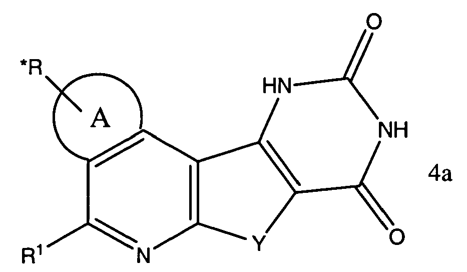 worin R¹, A, R* und Y dasselbe wie oben bedeuten.
   Bildung von Verbindungen der allgemeinenen Formel 4b, 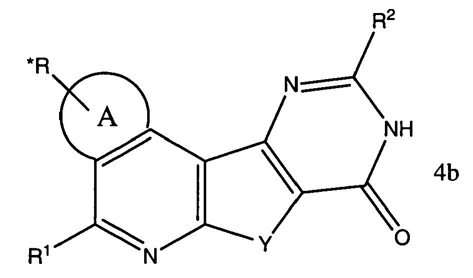
   worin R¹, A, R* und Y dasselbe wie oben bedeuten,
   durch Umsetzung der Verbindungen der allgemeinen Formel 3, mit R^{&}-Carbonsäure-chloriden und -bromiden (R^{§}-COOCI, R^{§}-COOBr), wobei R^{&} bedeutet:
   C₂₋₁₄Alkyl, C₃₋₁₄Cycloalkyl, C₂₋₁₄Alkenyl, C₃₋₁₄Cycloalkenyl,
   C₂₋₁₄Alkinyl,
   mono-, bi- oder tricyclische gesättigte oder ein- oder mehrfach
   ungesättigte heterocyclische Reste mit insgesamt 4-14 Ring-Atomen,
   davon 1-5 Heteroatomen, die vorzugsweise N, O, S und Se sind,
   ggf. ein- oder mehrfach gleich oder verschieden substituiert
   mit
      - OH, -SH, -O-C₁₋₈Alkyl, -OC₆₋₁₄Aryl, -S-C₁₋₄Alkyl, -S-C₆₋₁₄Aryl,
      - SO-C₁₋₄Alkyl, -SO-C₆₋₁₄Aryl, -SO₂-C₁₋₄Alkyl, -SO₂-C₆₋₁₄Aryl, -SO₃H,
      - OSO₂C₁₋₈Alkyl, -OSO₂C₆₋₁₄Aryl, -COOH, -COOC₁₋₈Alkyl, -(CO)C₁₋₈Alkyl,
      - COOH, -COOC₁₋₈Alkyl, -CONH₂, -CONHC₁₋₆Alkyl, -CON(C₁₋₆Alkyl)₂,
      - NH₂, -NHC₁₋₆Alkyl, -N(C₁₋₆Alkyl)₂, -NHC₆₋₁₄Aryl, -NH-Hetaryl,
      - N(C₆₋₁₄Aryl)₂, -N(C₁₋₆Alkyl)(C₆₋₁₄Aryl),
      - F, -Cl, -Br, -I, -CN, -NO₂, -SCN

      wobei ggf. vom Fachmann hinlänglich bekannte Schutzgruppentechniken (siehe Lit.: *T. W. Greene, P. G. M. Wufs, Protecting Groups In Organic Synthesis, John Wiley & Sons, 3. Auflage, 1999)* angewendet werden können,
   oder
   Umsetzung der Verbindungen der allgemeinen Formel 3, mit R&-Carbonsäureanhydriden (R^{§}-CO-O-CO-R^{§}), wobei R^{&} dasselbe wie oben bedeutet, jeweils im Überschuss und in der Siedehitze,
   gegebenenfalls zusammen mit Pyridin oder einem aprotischen, hochsiedenden Lösungsmittel, wie zum Beispiel Toluol oder Xylol,
- Behandlung des gebildeten Niederschlages mit wässerig-ethanolischer Natronlauge oder wässriger Ammoniak-Lösung unter Erwärmen, nachfolgendem Ansäuern mit verdünnter Salzsäure;
- Umsetzung der tetracyclischen Pyrimidin-2,4-dione der allgemeinen Formel 4a mit Phosphortrichlorid, Phosphorpentachlorid, Phosphoroxidchlorid oder vorzugsweise Dichlorphenylphosphinoxid bzw. deren Mischungen, jeweils in der Siedehitze,
   zu den tetracyclischen 2,4-Dichlorpyrimidinen der allgemeinen Formel 5, 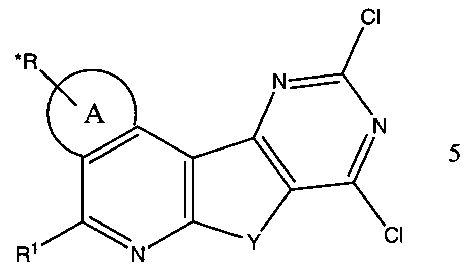 worin R¹, A, R* und Y dasselbe wie oben bedeuten;
- Umsetzung der tetracyclischen 2,4-Dichlorpyrimidine der allgemeinen Formel 5 mit Morpholin, Thiomorpholin, Thiomorpholino-S-Oxid, Thiomorpholin-S,S-Dioxid, Pyrrolidin, Piperidin, Homopiperazin, Piperazin, N-Alkylpiperazinen, C₁₋₆, N-Hydroxyalkylpiperazinen, C₁₋₃, N-Benzylpiperazin, oder N-Aryl-piperazinen,
   mit ein oder mehrfach, gleich oder ungleich am heterocycloaliphatischen Ring substituierten Aminen,
   mit weiteren sekundären, mono- oder polycyclischen cycloaliphatischen Aminen mit insgesamt 5-14 Ringatomen, einschließlich der am C-Skelett mit R^{§} ein oder mehrmals, gleich oder ungleich substituierten Vertreter,
   mit sekundären aliphatischen oder aromatischen Aminen, R⁵R⁶NH [worin R⁵ und R⁶ gleich oder ungleich sein können und C₁₋₆Alkyl, Benzyl, Phenyl, 1- und 2-Naphthyl, 2-, 3- und 4-Pyridyl, Chinolinyl, Isochinolinyl, 2-Thienyl, 2-Furyl usw. (wobei alle vorstehend genannten Reste auch mit R^{§} ein oder mehrmals, gleich oder ungleich substituiert sein können) bedeuten];
   mit primären Aminen, R⁵NH₂ (worin R⁵ dasselbe wie oben bedeutet),
   mit Natriumazid,
   mit Ammoniak, Hydroxylamin, O-C₁₋₃Alkylhydroxylaminen, N-C₁₋₃Alkylhydroxyl-aminen, N,N-Di (C₁₋₃)alkylhydroxylaminen, Hydrazin, C₁₋₄Alkyl- und Di-(C₁₋₄) alkylhydrazinen, Benzylhydrazinen, Carbonsäurehydraziden, N,N-Diacylhydrazinen,
   mit Harnstoff, N-Alkyl-, N,N-Dialkyl- und N,N'-Dialkylharnstoffen,
   mit Imidazol, 1,2,4-Triazol, Pyridin, Pyrazin, Pyridazin, einschliesslich der mit R^{§} substituierten Vertreter dieser Azaheterocyclen;
   mit Natrium- oder Kaliumalkoholaten von
   - C₁₋₄Alkanolen,
   - mit -OH, -SH, -SCH₃, -F, -Cl, -Br, -F₂, -Cl₂, -F₃ oder -Cl₃ substituierter C₁₋₄Alkanole,
   - C₂₋₅Alkenolen oder C₂₋₅Alkinolen,
   - C₄₋₇Cycloalkanolen und C₄₋₇Cycloalkenolen (ggf. am C- Skelett substituiert),
   mit Natrium- oder Kaliumphenolaten von mono-, bi- oder tricyclischen C₆₋₁₄ Aromaten oder Heteroaromaten,in Alkanolen oder gegebenenfalls aprotischen, dipolaren Lösungsmitteln unter Erwärmen, in Ausnahmefällen auch bei Raumtemperatur,
   zu den tetracyclischen 2-Chlor-pyrimidinen der allgemeinen Formeln 6a bzw. 6b, 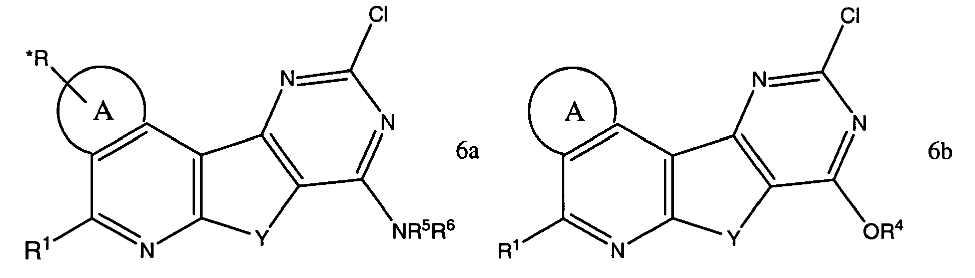
   worin R¹, R⁴, R⁵, R⁶ sowie A, R* und Y dasselbe wie oben bedeuten;
- Umsetzung der 2-Chlor-4-NR⁵R⁶-pyrimidine der allgemeinen Formel 6a,
   worin R¹, R⁴, R⁵, R⁶ sowie A, R* und Y dasselbe wie oben bedeuten,
   mit Natrium- oder Kaliumalkoholaten von
   - C₁₋₄Alkanolen,
   - mit -OH, -SH, -SCH₃, -F, -Cl, -Br, -F₂, -Cl₂, -F₃ oder -Cl₃ substituierter C₁₋₄Alkanole,
   - C₂₋₅Alkenolen oder C₂₋₅Alkinolen,
   - C₄₋₇Cycloalkanolen und C₄₋₇Cycloalkenolen (ggf. am C- Skelett substituiert),
      mit Natrium- oder Kaliumphenolaten von mono-, bi- oder tricyclischen C₆₋₁₄ Aromaten oder Heteroaromaten,

   in aprotischen, dipolaren Lösungsmitteln oder ggf. Alkanolen unter Erwärmen,
   zu den Verbindungen der allgemeinen Formel 1 a, worin R¹, R⁴, R⁵, R⁶ sowie A, R^{*} und Y dasselbe wie oben bedeuten;
- Umsetzung der 2-Chlor-4-OR⁴-pyrimidine der allgemeinen Formel 6b, worin R¹, R⁴, R⁵, R⁶ sowie A, R* und Y dasselbe wie oben bedeuten,
   mit Morpholin, Thiomorpholin, Thiomorpholino-S-Oxid, Thiomorpholin-S,S-Dioxid, Pyrrolidin, Piperidin, Homopiperazin, Piperazin, N-Alkylpiperazinen, C₁₋₆, N-Hydroxyalkylpiperazinen, C₁₋₃, N-Benzylpiperazin, oder N-Aryl-piperazinen,
   mit ein oder mehrfach, gleich oder ungleich am heterocycloaliphatischen Ring substituierten Aminen,
   mit weiteren sekundären, mono- oder polycyclischen cycloaliphatischen Aminen mit insgesamt 5-14 Ringatomen, einschließlich der am C-Skelett mit R^{§} ein oder mehrmals, gleich oder ungleich substituierten Vertreter,
   mit sekundären aliphatischen oder aromatischen Aminen, R⁵R⁶NH [worin R⁵ und R⁶ gleich oder ungleich sein können und C₁₋₆Alkyl, Benzyl, Phenyl, 1- und 2-Naphthyl, 2-, 3- und 4-Pyridyl, Chinolinyl, Isochinolinyl, 2-Thienyl, 2-Furyl usw. (wobei alle vorstehend genannten Reste auch mit R^{§} ein oder mehrmals, gleich oder ungleich substituiert sein können) bedeuten];
   mit primären Aminen, R⁵NH₂ (worin R⁵ dasselbe wie oben bedeutet),
   mit Natriumazid,
   mit Ammoniak, Hydroxylamin, O-C₁₋₃Alkylhydroxylaminen, N-C₁₋₃₋Alkylhydroxylaminen, N,N-Di(C₁₋₃)alkylhydroxylaminen, Hydrazin, C₁₋₄Alkyl- und Di-(C₁₋₄)alkylhydrazinen, Benzylhydrazinen, Carbonsäurehydraziden, N,N-Diacylhydrazinen, durch Erhitzen in aprotischen Lösungsmitteln unter Rückfluss zu den Verbindungen der allgemeinen Formel 1b, worin R¹, R⁴, R⁵, R⁶ sowie A, R* und Y dasselbe wie oben bedeuten;

### Untersuchungen zur PDE4A-Hemmung und Hemmung der TNFα-Freisetzung

### Methodenbeschreibung

Die Verbindungen der allgemeinen Formel 1 sind starke Inhibitoren der Phosphodiesterase 4 und gleichzeitig Inhibitoren der Freisetzung von TNFalpha. Die Verbindungen der allgemeinen Formeln 4a und 4b sind insbesondere starke Inhibitoren der Freisetzung von TNFalpha.

Die Volllängen-Formen der Phosphodiesterasen wurden aus zytosolischen Präparationen der humanen monozytischen Zellinien U937 oder HL60 erhalten und durch Anionen-Austauschchromatographie an einer Poros HQ Säule (starker Anionen-Austauscher) entsprechend einer adaptierten Methode von Lavan B. E., Lakey T., Houslay M. D. Biochemical Pharmacology, (1989), 38(22), 4123-4136., und Silver P. J et al., Eur. J. Pharmacol. (1988), 150: 85-94, and T. J. Torphy et al., J. Pharm. Exp. Ther. (1992), 263: 1195-1205 teilweise gereinigt.

Humane rekombinante PDE4A katalytische Domäne, die die Aminosäuren 342 - 704 umfasst, wurde in Escherichia coli kloniert und exprimiert, wie beschrieben durch W. Richter et al., Protein Expression and Purification (2000) 19: 375-383. Die anderen Phosphodiesterasen wurden entweder aus Zellinien humanen Ursprungs gewonnen (TPH1 Monozyten Linie für PDE1, MCF7 für PDE5), oder in SF9 Insektenzellen rekombinant exprimiert (für PDE3A, PDE3B, PDE7A und PDE4D), entsprechend einer adaptierten Methode von Luckow, V. A. et al., in: Recombinant DNA Technology&Applications., (1991) eds. Prokop, Bajpai, R. K.&Ho, C. S., pp 97-152.

Die Phosphodiesterase Aktivität wurde wie beschrieben durch den Hersteller Amersham Biosciences und durch Horton J.K and Baxendale P. M., Methods Mol. Biol. (1995) 41: 91-105 mittels des SPA-Assays bestimmt.

Die Reaktionsmischungen enthielten 100 mM Tris-HCl (pH 7.5), 5 mM MgCl₂, 3.8 mM 2-mercaptoethanol, und 1*µ*M cAMP oder cGMP als Substrat, die Inhibitoren in unterschiedlichen Konzentrationen und ferner weitere Komponenten, die für die Detektion der individuellen Isoenzyme benötigt wurden (s. unten). Die Reaktion wurde durch Zugabe des Enzyms gestartet. Das Arbeitsvolumen beträgt 100 *µ*l in den wells einer weißen 96-well Mikrotiterplatte. Die Testsubstanzen wurden als Stock-Lösungen in DMSO hergestellt. Die DMSO-Konzentration im Reaktionsansatz war 1 % v/v. Bei dieser DMSO-Konzentration wird die PDE-Aktivität nicht beeinträchtigt. Nach Start der Reaktion durch Zugabe von Enzym, wurden die Proben bei 22° C für 20 Minuten inkubiert. Die Reaktion wurde gestoppt durch Zugabe von SPA-Bead Suspension, die nach Hersteller vorgegebene Zinksulfatkonzentration enthält. Danach lässt man die Beads für 30 Minuten sedimentieren und wertet die Platten am Lumineszenz Mikrotiterplattenreader Fluostar Optima (BMG Labtechologies) aus.

[³H]-cAMP wurde als Substrat für die Bestimmung der Aktivität von PDE 2, 3, 4 und 7 eingesetzt und [³H]-cGMP für die Bestimmung der Aktivität von PDE 5. Die unspezifischen Enzymaktivitäten wurden in Anwesenheit von 100 *µ*M rolipram (für PDE 4) und in Anwesenheit von 100 *µ*M IBMX (für PDE 3 und 5) bestimmt und von den Testwerten subtrahiert. Die Inkubationsproben des PDE 3 Assays enthalten 10 *µ*M rolipram, um mögliche Kontaminationen durch PDE 4 zu inhibieren. PDE 2-Aktivität wird unter Verwendung eines SPA-Assays von Amersham Biosciences in Gegenwart des PDE 2 Aktivators 5 *µ*M cGMP getestet.

IC₅₀-Werte im Bereich von 10⁻⁹ bis 10⁻⁵ M wurden für die in der Erfindung beschriebenen Inhibitoren bezüglich ihrer inhibitorischen Wirkung auf Phosphodiesterase 4 beobachtet. Die Selektivität zu den PDE's 2, 3 und 5 weist einen Faktor von 100 bis 10 000 auf.

Die Stimulierung isolierter Leukozyten für die Freisetzung von Zytokinen kann auf verschiedenen Wegen erfolgen. Lipopolysaccharide (LPS) stellen einen Stimulus für die Untersuchung der Freisetzung von TNFα dar. LPS ist Bestandteil bakterieller Zellwände und wird beim Abtöten der Bakterien (durch Antibiotika oder das natürliche Immunsystem) freigesetzt. LPS stimuliert insbesondere die Aktivität phagozytierender Leukozyten (Gewebsmakrophagen, Granulozyten, Monozyten) und verursacht die Infiltration von Leukozyten vom peripheren Blut in das betroffene Gewebe. Ein Zytokin von besonderer Bedeutung für diese Mechanismen ist TNFα, das in großen Mengen durch die betroffenen Zellen sezerniert wird. Hauptquelle dabei sind Monozyten und Makrophagen. TNFα initiiert und prolongiert den Entzündungsprozess im Zusammenspiel mit anderen Mediatoren.

Für die Untersuchung des Effektes auf die LPS-induzierte TNFα-Freisetzung wurde eine Methode verwendet, die von Marx, D., Tassabehji, M., Heer, S., Hüttenbrink, K.-B., and I. Szelenyi, Pulmonary Pharmacology & Therapeutics (2002) 15, 7-15 beschrieben wurde. Die Methode in Kürze: Humanes Blut wurde von verschiedenen Spendern entnommen, durch Zusatz von 10 mM Na-Citrat ungerinnbar gemacht und 1:5 mit RPMI 1640 Zellkulturmedium verdünnt. Die Testsubstanzen wurden den Blutproben in verschiedenen Konzentrationen zugefügt. 15 Minuten später wurden die Leukozyten durch Zusatz von Lipopolysacchariden (LPS) aus Salmonella abortus equi in einer Endkonzentration von 1µg/ml stimuliert. Nach Inkubation der Testansätze für 24 Stunden bei 37°C. und unter 5% CO₂ in wassergesättigter Luft, wurde das Blut zentrifugiert und die Konzentration an TNFα im zellfreien Überstand unter Verwendung eines käuflichen ELISA (BD Biosciences) nach Angaben des Herstellers exakt vermessen.

Die nachfolgende Auflistung beinhaltet erfindungsgemäße Substanzen der allgemeinen Formel 1, die im PDE-4A-Hemmassay einen IC₅₀-Wert von <50 nM aufweisen:
10-Ethoxy-5-morpholino-8-piperazin-1-yl-pyrimido[4',5':4,5]thieno[2,3-c]-1,2,3,4-tetrahydro-isochinolin (Beispiel 2)
7-Ethoxy-2,3-dihydro-4-morpholino-9-piperazin-1 yl-1H-cyclopenta[4',5']pyrido[3',2':4,5]thieno[3,2-d]pyrimidin (Beispiel 7)
8-Ethoxy-5-piperidino-10-piperazin-1-yl-pyrimido[4',5':4,5]thieno[2,3-c]-1,2,3,4-tetrahydro-isochinolin-hydrochlorid (Beispiel 8)
8-Ethoxy-5-phenyl-10-piperazin-1-yl-pyrimido[4',5':4,5]thieno[2,3-c]-1,2,3,4-tetrahydro-isochinolin-hydrochlorid (Beispiel 13)
10-Ethoxy-5-phenyl-8-piperazin-1-yl-pyrimido[4',5':4,5]thieno[2,3-c]-1,2,3,4-tetrahydro-isochinolin (Beispiel 14)

Die nachfolgende Auflistung beinhaltet erfindungsgemäße Substanzen der allgemeinen Formeln 1, 4a und 4b, die im TNFα-Hemmassay einen IC₅₀-Wert zwischen 0,1 *µ*M bis <10 *µ*M aufweisen:
8-Ethoxy-5-morpholino-10-piperazin-1-yl-pyrimido[4',5':4,5]thieno[2,3-c]-1,2,3,4-tetrahydro-isochinolin (Beispiel 1)
1 0-Ethoxy-5-morpholino-8-piperazin-1-yl-pyrimido[4',5':4,5]thieno[2,3-c]-1,2,3,4-tetrahydro-isochinolin (Beispiel 2)
7-Ethoxy-2,3,4,5-tetrahydro-4-morpholino-9-piperazin-1 yl-1 H-cyclohepta[4',5']pyrido[3',2':4,5]thieno[3,2-d]pyrimidin (Beispiel 4)
8-Ethoxy-5-piperidino-10-piperazin-1-yl-pyrimido[4',5':4,5]thieno[2,3-c]-1,2,3,4-tetrahydro-isochinolin-hydrochlorid (Beispiel 8)
5-Phenyl-pyrimido[4',5':4,5]thieno[2,3-c]-1,2,3,4-tetrahydro-isochinolin-8,10(9H,11H)-dion (Beispiel 15)

Die Erfindung wird durch die nachfolgenden Beispiele näher erläutert:

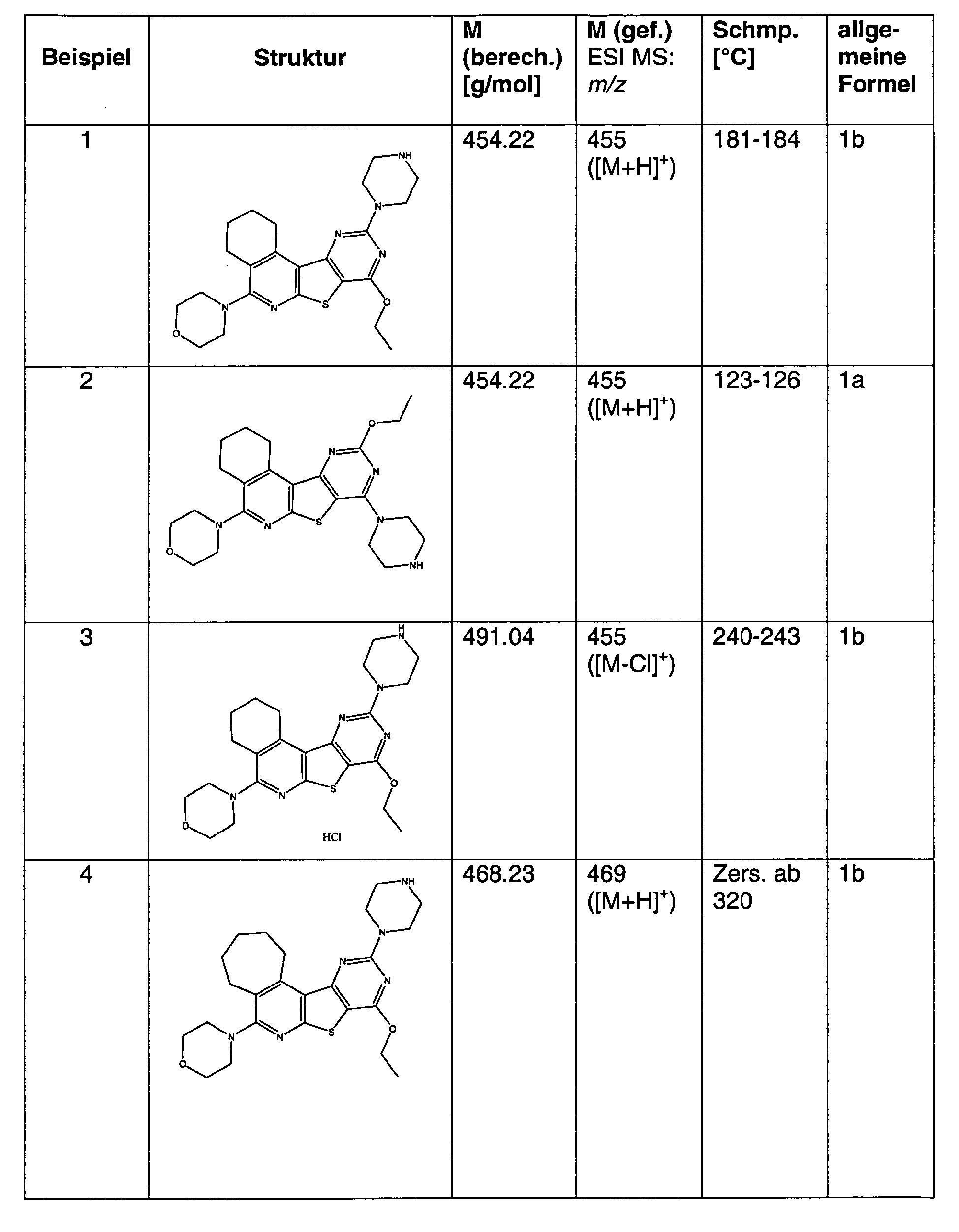

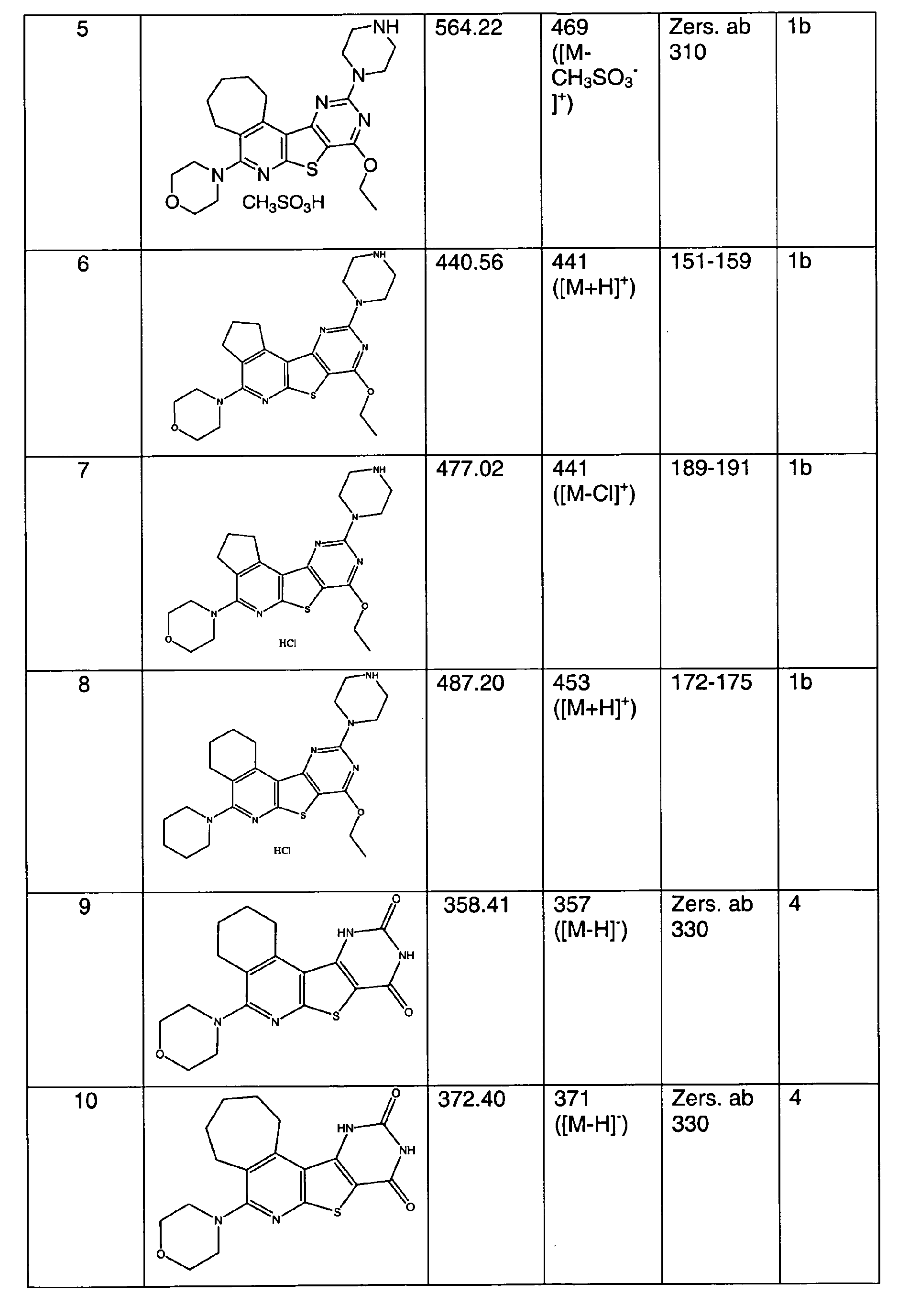

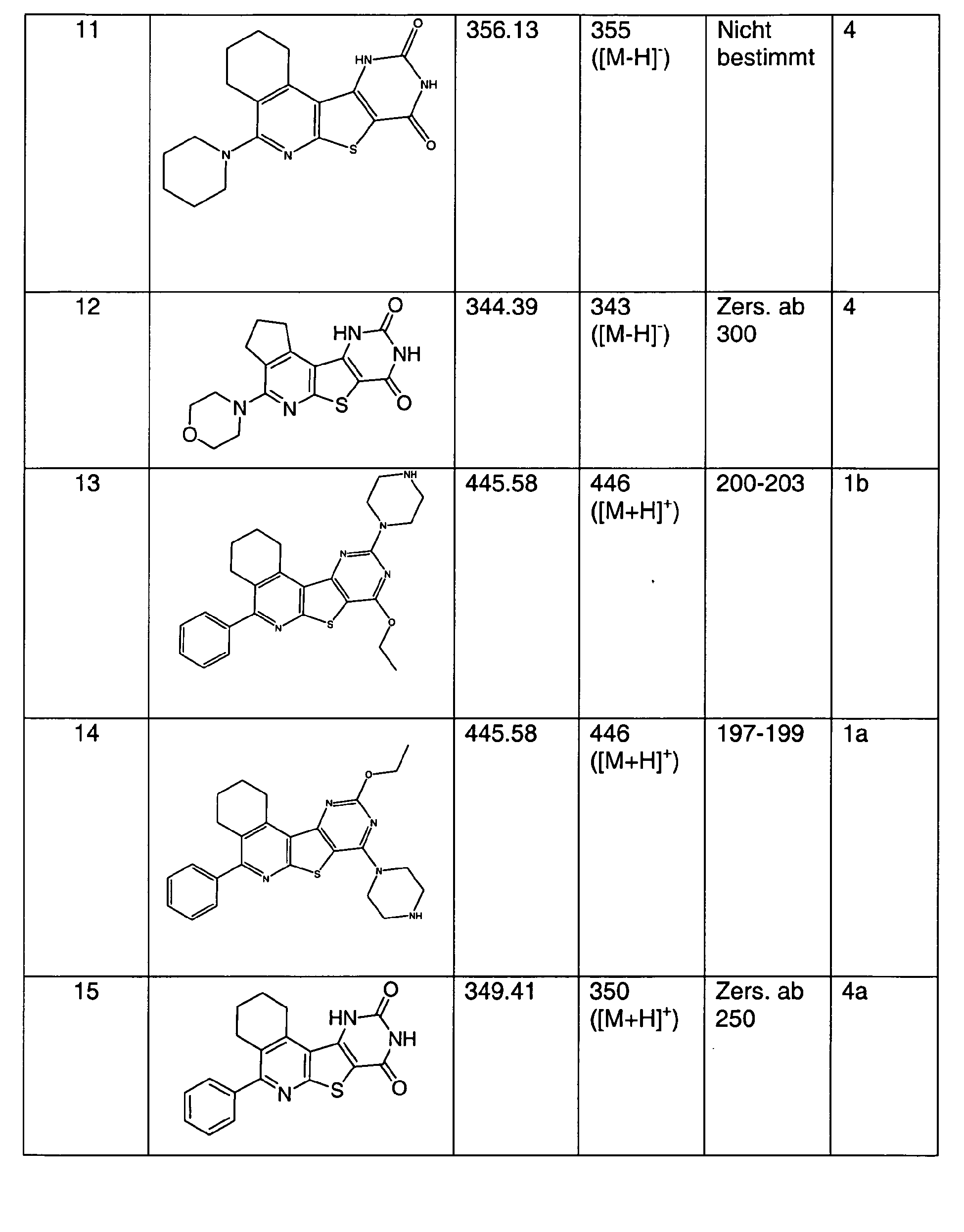

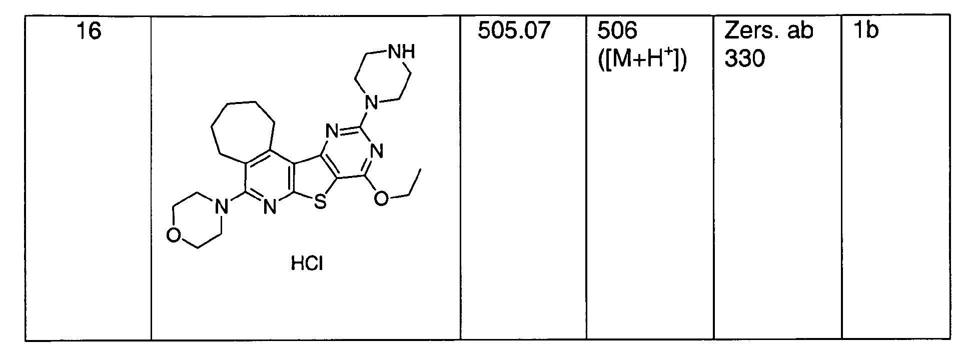

Die Synthese der angeführten Beispiele wird exemplarisch für die Beispiele 1, 2, 3 (jeweils allgemeine Formel 1) und 9 (allgemeine Formel 4a) erläutert:

Die Verbindungen der allgemeinen Formel 2 werden nach folgender Lit. hergestellt:
1) *E. G. Baronikyan, A. S. Noravyan, Hayastani Kimiakan Handes (2002), 55(4), 67-71*
*2) K. Gewald, M. Hentschel, Ger. (East) (1974), DD 105805*

### 1-Amino-5-morpholino-thieno[2,3-c]-6,7,8,9-tetrahydro-isochinolin-2-carbonsäureamid (allgemeine Formel 3)

4.00 g (20 mmol) 3-Mercapto-1-morpholino-5,6,7,8-tetrahydroisochinolin-4-carbonitril (allgemeine Formel 2) und 1.80 g (20 mmol) 2-Chloracetamid werden in 50 ml Ethanol suspendiert und mit 1.50 g (40 mmol) Natriumethylat versetzt. Das Reaktinonsgemisch wird 3 h unter Rückfluss gehalten. Es wird filtriert und mit Ethanol, Wasser gewaschen und getrocknet.
Ausbeute: 4.00 g (80 %);

### 5-Morpholino-pyrimido[4',5':4,5]thieno[2,3-c]-1,2,3,4-tetrahydro-isochinolin-8,10(9H,11H)-dion (Beispiel 9, allgemeine Formel 4a)

3.0 g (9 mmol) 1-Amino-5-morpholino-thieno[2,3-c]-6,7,8,9-tetrahydro-isochinolin-2-carbonsäureamid (allgemeine Formel 3) werden in 100 ml Dioxan suspendiert, anschließend werden 1.10 ml (8 mmol) Chlorameisensäure-trichlormethylester zugegeben und 3 h unter Rückfluss erhitzt. Der Niederschlag wird filtriert und getrocknet.
Ausbeute: 3.00 g (93%);
ESI MS: m/z = 359 ([M+H]⁺)

### 8,10-Dichlor-5-morpholino-pyrimido[4',5':4,5]thieno[2,3-c]-1,2,3,4-tetrahydroisochinolin (allgemeine Formel 5)

3.0 g (8 mmol) 5-Morpholino-pyrimido[4',5':4,5]thieno[2,3-c]-1,2,3,4-tetrahydroisochinolin-8,10(9H,11H)-dion (Beispiel 9, allgemeine Formel 4a) und 20 ml (140 mmol) Dichlorphenylphosphinoxid werden 6 h bei 193°C erhitzt. Anschließend wird auf 40 g Eiswasser gegossen und mit gesättigter Natriumhydrogencarbonat-Lösung neutralisiert. Der Niederschlag wird abgesaugt, mit 100 ml Wasser gewaschen und getrocknet.
Ausbeute: 2.00 g (63 %),
ESI MS: *m*/*z* = 395 ([M+H]⁺)

### 10-Chlor-8-ethoxy-5-morpholino-pyrimido[4',5':4,5]thieno[2,3-c]-1,2,3,4-tetrahydro-isochinolin (allgemeine Formel 6b)

0.50 g (1 mmol) 8,10-Dichlor-5-morpholino-pyrimido[4',5':4,5]thieno[2,3-c]-1,2,3,4-tetrahydro-isochinolin (allgemeine Formel 5) werden in 40 ml Ethanol suspendiert und mit 0.12 g (2 mmol) NaOEt versetzt. Das Gemisch wird 24 h bei RT gerührt. Anschließend wird der Niederschlag abgesaugt, mit Ethanol gewaschen und getrocknet.
Ausbeute: 3.90 g (96 %),
ESI MS: m/z = 405 ([M+H]⁺)

### 10-Chlor-8-piperazin-1-yl-5-morpholino-pyrimido[4',5':4,5]thieno[2,3-c]-1,2,3,4-tetrahydro-isochinolin (allgemeine Formel 6a)

0.30 g (0.76 mmol) 8,10-Dichlor-5-morpholino-pyrimido[4',5':4,5]thieno[2,3-c]-1,2,3,4-tetrahydro-isochinolin (allgemeine Formel 5) und (1 mmol) Piperazin werden in 40 ml Tetrahydrofuran 6 h bei RT gerührt. Anschließend wird das Lösungsmittel entfernt, der Rückstand in Wasser suspendiert, abgesaugt, mit ca. 40 ml Wasser gewaschen und getrocknet.
Ausbeute: 0.3 g (89 %)
ESI MS: m/z = 444 ([M+H]⁺)

### 8-Ethoxy-5-morpholino-10-piperazin-1-yl-pyrimido[4',5':4,5]thieno[2,3-c]-1,2,3,4-tetrahydro-isochinolin (Beispiel 1, allgemeine Formel 1b)

0.10 g (0.25 mmol) 10-Chlor-8-ethoxy-5-morpholino-pyrimido[4',5':4,5]thieno[2,3-c]-1,2,3,4-tetrahydro-isochinolin (allgemeine Formel 6b) und 0.12g (1.4 mmol) Piperazin werden in 20 ml Toluol 4 h unter Rückfluss erhitzt. Anschließend wird das Lösungsmittel entfernt und der Rückstand in Dichlormethan suspendiert. Die organische Phase wird mit gesättigter Natriumcarbonat-Lösung gewaschen, über Natriumsulfat getrocknet und das Lösungsmittel entfernt. Der Rückstand wird durch Flash-Chromatographie (Kieselgel, Chloroform/Ethanol 10:2) gereinigt.
Ausbeute: 0.11 g (97 %)
ESI MS: *m*/*z* = 444 ([M+H]⁺)

### 10-Ethoxy-5-morpholino-8-piperazin-1-yl-pyrimido[4',5':4,5]thieno[2,3-c]-1,2,3,4-tetrahydro-isochinolin (Beispiel 2, allgemeine Formel 1a)

0.30 g (0.44 mmol) 10-Chlor-8-piperazin-1-yl-5-morpholino-pyrimido[4',5':4,5]thieno-[2,3-c]-1,2,3,4-tetrahydro-isochinolin (allgemeine Formel 6a) und 0.30 g (4.40 mmol) Natriumethylat werden in 10 ml Ethanol 3 h unter Rückfluss erhitzt. Anschließend wird das Lösungsmittel entfernt und der Rückstand in wenig Wasser suspendiert. Die wässrige Suspension wird mit Dichlormethan extrahiert, die organische Phase über Natriumsulfat getrocknet und das Lösungsmittel entfernt.
Ausbeute: 0.21 g (97%)
ESI MS: *m*/*z* = 454.22 ([M+H]⁺)

### 8-Ethoxy-5-morpholino-10-piperazin-1-yl-pyrimido[4',5':4,5]thieno[2,3-c]-1,2,3,4-tetrahydro-isochinolin-hydrochlorid (Beispiel 3, allgemeine Formel 1 b)

0.28 g (0.62 mmol) 8-Ethoxy-5-morpholino-10-piperazin-1-yl-pyrimido[4',5':4,5]thieno-[2,3-c]-1,2,3,4-tetrahydro-isochinolin (Beispiel 1, allgemeine Formel 1b) werden in 2 ml Dichlormethan gelöst. Es werden 540 µl (0.68 mmol) 1.25 M ethanolische Salzsäure zugegeben und 2 min gerührt. Anschließend wird das Lösungsmittel im Vak. entfernt und der erhaltene Rückstand abgesaugt und getrocknet.
Ausbeute: 0.21 g (69 %)
ESI MS: m/z = 454.22 ([M+H]⁺)

## Patentansprüche

1. Substituierte anellierte Pyrido[3',2':4,5]thieno[3,2-d]pyrimidine (Y=S) und anellierte Pyrido[3',2':4,5]furo[3,2-d]pyrimidine (Y=O) der allgemeinen Formel 1 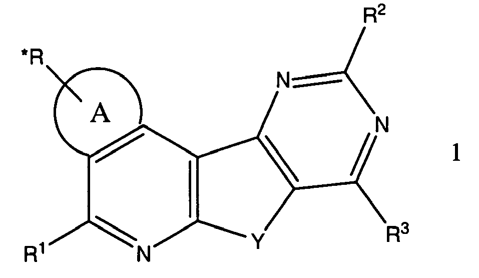
Y
- Schwefel oder Sauerstoff,
A
- anellierter Carbocyclus mit 5 bis 8 Ringatomen, einfach oder mehrfach ungesättigt sowie aromatisch, (ggf. mit R* substituiert und/oder ggf. mit R^{§} substituiert);
- anellierter Bicyclus mit 6 bis 11 Ringatomen, einfach oder mehrfach ungesättigt, welcher auch Stickstoff, Sauerstoff und/oder Schwefel enthalten kann (ggf. mit R* substituiert und/ oder ggf. mit R^{§} substituiert);
- annelierter Heterocyclus mit 5 bis 8 Ringatomen einfach oder mehrfach ungesättigt sowie aromatisch, welcher Stickstoff, Sauerstoff und Schwefel einfach, mehrfach, gleich oder ungleich unabhängig voneinander im Cyclus enthalten kann (ggf. mit R* substituiert und/oder ggf. mit R^{§} substituiert),
R¹
- Wasserstoff (außer wenn A ein anellierter unsubstituierter Cyclopenten-Ring kombiniert mit R²,R³ gleich -OH ist)
- C₁₋₁₀Alkyl (ggf. mit R^{§} substituiert),
- C₂₋₁₂ Alkenyl und C₂₋₁₂ Alkinyl (jeweils ggf. mit R^{§} substituiert),
- Difluormethyl, Trifluormethyl,
- Benzyl, Phenyl-(C₂-₆)alkyl (jeweils ggf. mit R^{§} substituiert), Phenyl (ggf. mit R^{§} substituiert), dabei inbesondere: 4-R^{§} -Phenyl, 3-R^{§} -Phenyl, 2-R^{§}-Phenyl, 3-R^{§},4-R^{§}-Phenyl, 3-R^{§},4-R^{§}-,5-R^{§}-Phenyl, 2-R^{§}, 4-R^{§}-Phenyl,
- 1-Naphthyl, 2-Naphthyl (jeweils ggf. mit R^{§} substituiert),
- C₃₋₁₄Cycloalkyl, C₃₋₁₄Cycloalkenyl (jeweils ggf. mit R^{§}-substituiert),
- mono- oder bicyclische gesättigte oder ein- oder mehrfach ungesättigte Heterocyclen mit 5 - 14 Ringatomen darunter 1 - 4 Heteroatome, die vorzugsweise N, O und S sind,
- C₂₋₁₂Alkylacyl (ggf. mit R^{§} substituiert),
- Benzoyl, 1- und 2-Naphthoyl (jeweils ggf. mit R^{§} substituiert),
- Heterocyclylacyl [z.B. Nicotinoyl, Isonicotinoyl, 2-Picolinoyl, 2-Thienoyl, 2-Furoyl] (ggf. mit R^{§} substituiert)
- Hydroxy,
- Sulfhydryl,
- C₁₋₁₀ Alkoxy,
- Alkylthio, Alkylsulfinyl und Alkylsulfonyl (jeweils C₁₋₆)
- Formyl, Carboxyl, C₁₋₄Alkoxycarbonyl;
- CONH₂, CONHAlk und CONAlk₂ (mit "Alk" jeweils C₁₋₆),
- Cyano, Rhodano, Nitro, SO₃H, SO₂OAlk (mit "Alk": C₁₋₅),
- Chlor, Brom, lod, Fluor,
- Amino, C₁₋₆Alkylamino, Di(C₁₋₅)alkylamino (jeweils ggf. mit R^{§} am Alkylrest substituiert),
- Morpholino, Thiomorpholino, Thiomorpholino-S-Oxid, Thiomorpholino-S,S-Dioxid, Pyrrolidino,
- Piperidino, 1-Piperazino, 4-Methyl-1-piperazino, 4-Hydroxyethyl-1-piperazino, 4-Phenyl-1-piperazino,
- Cycloalkylamino, C₃₋₁₄ Arylamino und Heteroarylamino [z.B. Phenyl-, 1- und 2-Naphthyl-, 2-, 3- oder 4-Pyridyl-, Chinolinyl-, Isochinolinyl-, Acridinyl-, Phenothiazinyl-, 2-Thienyl- und 2-Furylamino] (ggf. jeweils an den carbo- bzw. heterocyclischen Ringen mit R^{§} substituiert);
R²
- C₂₋₁₄Alkyl, C₃₋₁₄Cycloalkyl, C₂₋₁₄Alkenyl, C₃₋₁₄Cycloalkenyl, C₂₋₁₄Alkinyl (jeweils ggf. am C-Skelett der vorgenannten Reste mit R^{§} substituiert);
- Benzyl, Phenyl-(C₂₋₆)alkyl (jeweils ggf. mit R^{§} substituiert), Phenyl (ggf. mit R^{§} substituiert), dabei inbesondere: 4-R^{§} -Phenyl, 3-R^{§} -Phenyl, 2-R^{§}-Phenyl, 3-R^{§},4-R^{§}-Phenyl, 3-R^{§},4-R^{§}-,5-R^{§}-Phenyl, 2-R^{§}, 4-R^{§}-Phenyl,
- 1-Naphthyl, 2-Naphthyl (jeweils ggf. mit R^{§} substituiert);
- S-Alkyl (C₃-C₈), SO-Alkyl, SO₂-Alkyl, (jeweils C₁-C₈), S-Alkenyl, SO-Alkenyl, SO₂-Alkenyl (jeweils C₂-C₈) S-Alkinyl, SO-Alkinyl, SO₂-Alkinyl (jeweils C₂-C₆) (ggf. jeweils am C-Skelett der vorgenannten Reste mit -OH, -CN, - SCN, -NO₂, Phenyl oder C₃ - C₇Cycloalkyl substituiert);
- mono-, bi- oder tricyclische gesättigter oder ein- oder mehrfach ungesättigter heterocyclischer Rest mit insgesamt 4-14 Ring-Atomen, davon 1-5 Heteroatomen, die vorzugsweise N, O, S und Se sind (jeweils ggf. mit R^{§} substituiert);
- OR⁴, worin R⁴ bedeutet
- Wasserstoff
- CH₃, C₂H₅, (CH₂)₂CH₃, (CH₂)₃CH₃, CH(CH₃)₂, CH₂CH(CH₃)₂, C(CH₃)₃, CH₂CH₂OH, CH₂CH₂SH, CH₂CH₂SCH₃, CH₂CF₃, CH₂CCl₃, CH₂CHF₂, CH₂CHCl₂, CH₂CH₂F, CH₂CH₂Cl, CH₂CH₂Br,
- C₃₋₇Cycloalkyl [z.B.Cylopropyl, Cylopropylmethyl, Cylobutyl, Cyclobutylmethyl, Cyclopentyl, Cyclopentylmethyl, Cylohexyl, Cyclohexylmethyl] (ggf. am C- Skelett mit R^{§} substituiert),
- C₂₋₅Alkenyl und C₂₋₅Alkinyl,
- C₃₋₇ Cycloalkenyl,
- Aryl und Heteroaryl als Reste mono-, bi- oder tricyclischer Aromaten oder Heteroaromaten mit ggf. 6-14 Ring-Atomen [z.B. Phenyl, 4-R^{§} -Phenyl, 3-R^{§} -Phenyl, 2-R^{§}-Phenyl, 3-R^{§},4-R^{§}-Phenyl, 3-R^{§},4-R^{§}-,5-R^{§}-Phenyl],
- 1-Naphthyl, 2-Naphthyl (ggf. jeweils mit R^{§} substituiert),
- Pyridyl, Isochinolinyl, Chinolinyl, Acridinyl;
- NR⁵R⁶, worin dieser Substituent insgesamt bedeutet:
- Morpholino, Thiomorpholino, Thiomorpholino-S-Oxid Thiomorpholino-S,S-Dioxid, Pyrrolidino, Piperidino, 1-Piperazino, 1-Homopiperazino, 4-C₁₋₆₋Alkyl-1-piperazino, 4-(2-Hydroxyethyl)-1-piperazino, 4-Benzyl-1-piperazino, 4-Aryl-1-piperazino (ggf. mit R^{§} am heterocycloaliphatischen Ring substituiert),
- weitere Amino-Reste sekundärer, mono- oder polycyclischer cycloaliphatischer Amine mit insgesamt 5-14 Ringatomen, einschließlich der am C-Skelett mit R^{§} substituierte Vertreter;
- Amino-Reste sekundärer aliphatischer und aromatischer Amine, R⁵R⁶NH,
worin R⁵ und R⁶ unabhängig voneinander gleich oder ungleich sein können und bedeuten:
- C₁₋₆Alkyl, Benzyl, Phenyl, 1- und 2-Naphthyl, 2-, 3- und 4-Pyridyl, Chinolinyl, Isochinolinyl, 2-Thienyl, 2-Furyl (ggf. jeweils mit R^{§} substituiert);
- Aminoreste primärer Amine, R⁵NH₂, (worin R⁵ dasselbe wie oben bedeutet),
- Amino, NH₂, mit der Einschränkung, dass dann R³ nur die Bedeutung von OR⁴ hat ;
R³
- OR⁴, wobei R⁴ dasselbe wie oben bedeutet,
- NR⁵R⁶, mit gleicher Bedeutung wie oben
- Azido, Hydroxylamino, O-(C₁₋₃)Alkylhydroxylamino,
- N-(C₁₋₃)Alkylhydroxylamino, N,N-Di(C₁₋₃)alkylhydroxylamino,
- Hydrazino, (C₁₋₄)Alkyl- und Di(C₁₋₄)alkylhydrazino,
- Benzylhydrazino, Acylhydrazino, N,N-Diacylhydrazino,
- Carbamoylamino,
- 1-Imidazolyl, 1,2,4-Triazol-lyl, 1-Pyridinium,
- 1-Pyrazinium, 1-Pyridazinium, einschliesslich der alkylsubstituierten Vertreter dieser Azaheterocyclen;
R^{§}
- OH, -SH, -O-C₁-₈Alkyl, -O-C₆₋₁₄Aryl, -S-C₁-₄Alkyl, -S-C₆₋₁₄Aryl,
- SO-C₁₋₄Alkyl, -SO-C₆₋₁₄Aryl, -SO₂-C₁₋₄Alkyl, -SO₂-C₆₋₁₄Aryl, -SO₃H,
- OSO₂C₁₋₈Alkyl, -OSO₂C₆₋₁₄Aryl, -COOH, -COOC₁₋₈Alkyl, -(CO)C₁₋₈Alkyl,
- COOH, -CONH₂, -CONHC₁₋₆Alkyl, -CON(C₁₋₆Alkyl)₂,
- NH₂, -NHC₁₋₆Alkyl, -N(C₁₋₆Alkyl)₂, -NHC₆₋₁₄Aryl, -NH-Hetaryl,
- N(C₆₋₁₄Aryl)₂, -N(C₁₋₆Alkyl)(C₆₋₁₄Aryl),
- CH₃, -CHF₂, -CF₃, -C₂H₅, -C(CH₃)₂, -(CH₂) ₂CH₃, -(CH₂) ₃CH₃, -CH₂CH₂OH,
- CH₂CH₂SH, -CH₂CH₂SCH₃, -CH₂CF₃, -CH₂CCl₃, -CH₂CHF₂, -CH₂CHCl₂,
- CH₂CH₂F, -CH₂CH₂Cl, -CH₂CH₂Br, -Cylopropyl, -Cylopropylmethyl,
- Cylobutyl, -Cyclobutylmethyl, -Cyclopentyl, -Cyclopentylmethyl,
- Cylohexyl, -Cyclohexylmethyl,
- F, -Cl, -Br, -l, -CN, -NO₂, und -SCN.
R^{*} =O, =S, =N-H, =N-C₁₋₈Alkyl, =N-Aryl, =N-OH, =N-O-C₁₋₈A1kyl, =N-O-C₆₋₁₄Aryl
sowie pharmazeutisch verträgliche Salze, Solvate, aktive Metabolite, Tautomere und Prodrugs dieser Verbindungen,

2. Substituierte anellierte Pyrido[3',2':4,5]thieno[3,2-d]pyrimidin-2,4-dione bzw. -4-one (Y=S) und anellierte Pyrido[3',2':4,5]furo[3,2-d]pyrimidin-2,4-dione bzw. 4-one (Y=O) der allgemeinen Formeln 4a bzw. 4b 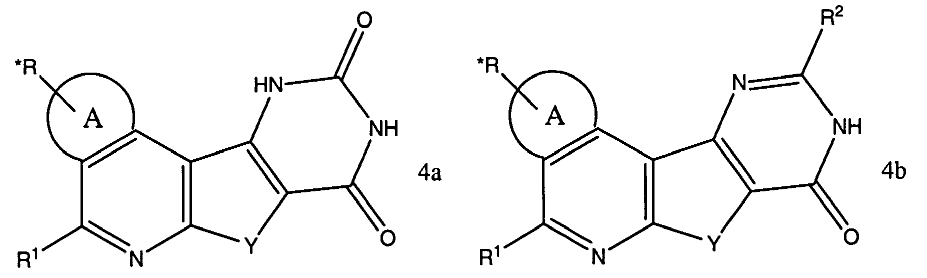
Y
- Schwefel oder Sauerstoff,
A
- anellierter Carbocyclus mit 5 bis 8 Ringatomen, einfach oder mehrfach ungesättigt sowie aromatisch, (ggf. mit R^{*} substituiert und/oder ggf. mit R^{§} substituiert);
- anellierter Bicyclus mit 6 bis 11 Ringatomen, einfach oder mehrfach ungesättigt, welcher auch Stickstoff, Sauerstoff und/oder Schwefel enthalten kann (ggf. mit R* substituiert und/oder ggf. mit R^{§} substituiert);
- annelierter Heterocyclus mit 5 bis 8 Ringatomen einfach oder mehrfach ungesättigt sowie aromatisch, welcher Stickstoff, Sauerstoff und Schwefel einfach, mehrfach, gleich oder ungleich unabhängig voneinander im Cyclus enthalten kann (ggf. mit R^{*} substituiert und/oder ggf. mit R^{§} substituiert),
R¹
- Wasserstoff (außer wenn A ein anellierter unsubstituierter Cyclopenten-Ring kombiniert mit R²,R³ gleich -OH ist)
- C₁₋₁₀Alkyl (ggf. mit R^{§} substituiert),
- C₂₋₁₂ Alkenyl und C₂₋₁₂ Alkinyl (jeweils ggf. mit R^{§} substituiert ),
- Difluormethyl, Trifluormethyl,
- Benzyl, Phenyl-(C₂₋₆)alkyl (jeweils ggf. mit R^{§} substituiert), Phenyl (ggf. mit R^{§} substituiert), dabei inbesondere: 4-R^{§} -Phenyl, 3-R^{§} -Phenyl, 2-R^{§}-Phenyl, 3-R^{§},4-R^{§}-Phenyl, 3-R^{§},4-R^{§}-,5-R^{§}-Phenyl, 2-R^{§}, 4-R^{§}-Phenyl,
- 1-Naphthyl, 2-Naphthyl (jeweils ggf. mit R^{§} substituiert),
- C₃₋₁₄Cycloalkyl, C₃₋₄Cycloalkenyl (jeweils ggf. mit R^{§}-substituiert),
- mono- oder bicyclische gesättigte oder ein- oder mehrfach ungesättigte Heterocyclen mit 5 - 14 Ringatomen darunter 1 - 4 Heteroatome, die vorzugsweise N, O und S sind,
- C₂₋₁₂Alkylacyl (ggf. mit R^{§} substituiert),
- Benzoyl, 1- und 2-Naphthoyl (jeweils ggf. mit R^{§} substituiert),
- Heterocyclylacyl [z.B. Nicotinoyl, Isonicotinoyl, 2-Picolinoyl, 2-Thienoyl, 2-Furoyl] (ggf. mit R^{§} substituiert)
- Hydroxy,
- Sulfhydryl,
- C₁₋₁₀ Alkoxy,
- Alkylthio, Alkylsulfinyl und Alkylsulfonyl (jeweils C₁₋₆)
- Formyl, Carboxyl, C₁₋₄Alkoxycarbonyl;
- CONH₂, CONHAlk und CONAlk₂ (mit "Alk" jeweils C₁₋₆),
- Cyano, Rhodano, Nitro, SO₃H, SO₂OAlk (mit "Alk": C₁₋₅),
- Chlor, Brom, lod, Fluor,
- Amino, C₁₋₆Alkylamino, Di(C₁₋₅)alkylamino (jeweils ggf. mit R^{§} am Alkylrest substituiert),
- Morpholino, Thiomorpholino, Thiomorpholino-S-Oxid, Thiomorpholino-S,S-Dioxid, Pyrrolidino,
- Piperidino, 1-Piperazino, 4-Methyl-1-piperazino, 4-Hydroxyethyl-1-piperazino, 4-Phenyl-1-piperazino,
- Cycloalkylamino, C₃₋₁₄ Arylamino und Heteroarylamino [z.B. Phenyl-, 1- und 2-Naphthyl-, 2-, 3- oder 4-Pyridyl-, Chinolinyl-, Isochinolinyl-, Acridinyl-, Phenothiazinyl-, 2-Thienyl- und 2-Furylamino] (ggf. jeweils an den carbo- bzw. heterocyclischen Ringen mit R^{§} substituiert);
R²
- C₂₋₁₄Alkyl, C₃₋₁₄Cycloalkyl, C₂₋₁₄Alkenyl, C₃₋₁₄Cycloalkenyl, C₂₋₁₄Alkinyl (jeweils ggf. am C-Skelett der vorgenannten Reste mit R^{§} substituiert);
- Benzyl, Phenyl-(C₂₋₆)alkyl (jeweils ggf. mit R^{§} substituiert), Phenyl (ggf. mit R^{§} substituiert), dabei inbesondere: 4-R^{§} -Phenyl, 3-R^{§} -Phenyl, 2-R^{§}-Phenyl, 3-R^{§},4-R^{§}-Phenyl, 3-R^{§},4-R^{§}-,5-R^{§}-Phenyl, 2-R^{§}, 4-R^{§}-Phenyl,
- 1-Naphthyl, 2-Naphthyl (jeweils ggf. mit R^{§} substituiert);
- S-Alkyl (C₃-C₈), SO-Alkyl, SO₂-Alkyl (jeweils C₁-C₈), S-Alkenyl, SO-Alkenyl, SO₂-Alkenyl (jeweils C₂-C₈) S-Alkinyl, SO-Alkinyl, SO₂-Alkiny (jeweils C₂-C₆) (ggf. jeweils am C-Skelett der vorgenannten Reste mit -OH, -CN, - SCN, -NO₂, Phenyl oder C₃ - C₇Cycloalkyl substituiert);
- mono-, bi- oder tricyclische gesättigter oder ein- oder mehrfach ungesättigter heterocyclischer Rest mit insgesamt 4-14 Ring-Atomen, davon 1-5 Heteroatomen, die vorzugsweise N, O, S und Se sind (jeweils ggf. mit R^{§} substituiert);
- OR⁴, worin R⁴ bedeutet
- Wasserstoff
- CH₃, C₂H₅, (CH₂)₂CH₃, (CH₂)₃CH₃, CH(CH₃)₂, CH₂CH(CH₃)₂, C(CH₃)₃, CH₂CH₂OH, CH₂CH₂SH, CH₂CH₂SCH₃, CH₂CF₃, CH₂CCl₃, CH₂CHF₂, CH₂CHCl₂, CH₂CH₂F, CH₂CH₂Cl, CH₂CH₂Br,
- C₃₋₇Cycloalkyl [z.B.Cylopropyl, Cylopropylmethyl, Cylobutyl, Cyclobutylmethyl, Cyclopentyl, Cyclopentylmethyl, Cylohexyl, Cyclohexylmethyl] (ggf. am C- Skelett mit R^{§} substituiert),
- C₂₋₅Alkenyl und C₂₋₅Alkinyl,
- C₃₋₇ Cycloalkenyl,
- Aryl und Heteroaryl als Reste mono-, bi- oder tricyclischer Aromaten oder Heteroaromaten mit ggf. 6-14 Ring-Atomen [z.B. Phenyl, 4-R^{§} -Phenyl, 3-R^{§} -Phenyl, 2-R^{§}-Phenyl, 3-R^{§},4-R^{§}-Phenyl, 3-R^{§},4-R^{§}-,5-R^{§}-Phenyl],
- 1-Naphthyl, 2-Naphthyl (ggf. jeweils mit R^{§} substituiert),
- Pyridyl, Isochinolinyl, Chinolinyl, Acridinyl;
- NR⁵R⁶, worin dieser Substituent insgesamt bedeutet:
- Morpholino, Thiomorpholino, Thiomorpholino-S-Oxid Thiomorpholino-S,S-Dioxid, Pyrrolidino, Piperidino, 1-Piperazino, 1-Homopiperazino, 4-C₁₋₆-Alkyl-1-piperazino, 4-(2-Hydroxyethyl)-1-piperazino, 4-Benzyl-1-piperazino, 4-Aryl-1-piperazino (ggf. mit R^{§} am heterocycloaliphatischen Ring substituiert),
- weitere Amino-Reste sekundärer, mono- oder polycyclischer cycloaliphatischer Amine mit insgesamt 5-14 Ringatomen, einschließlich der am C-Skelett mit R^{§} substituierte Vertreter;
- Amino-Reste sekundärer aliphatischer und aromatischer Amine, R⁵R⁶NH,
worin R⁵ und R⁶ unabhängig voneinander gleich oder ungleich sein können und bedeuten:
- C₁-₆Alkyl, Benzyl, Phenyl, 1- und 2-Naphthyl, 2-, 3- und 4-Pyridyl, Chinolinyl, Isochinolinyl, 2-Thienyl, 2-Furyl (ggf. jeweils mit R^{§} substituiert);
- Aminoreste primärer Amine, R⁵NH₂, (worin R⁵ dasselbe wie oben bedeutet),
- Amino, NH₂, mit der Einschränkung, dass dann R³ nur die Bedeutung von OR⁴ hat ;
R³
- OR⁴, wobei R⁴ dasselbe wie oben bedeutet,
- NR⁵R⁶, mit gleicher Bedeutung wie oben
- Azido, Hydroxylamino, O-(C₁₋₃)Alkylhydroxylamino,
- N-(C₁₋₃)Alkylhydroxylamino, N,N-Di(C₁₋₃)alkylhydroxylamino,
- Hydrazino, (C₁₋₄)Alkyl- und Di(C₁₋₄)alkylhydrazino,
- Benzylhydrazino, Acylhydrazino, N,N-Diacylhydrazino,
- Carbamoylamino,
- 1-Imidazolyl, 1,2,4-Triazol-1yl, 1-Pyridinium,
- 1-Pyrazinium, 1-Pyridazinium, einschliesslich der alkylsubstituierten Vertreter dieser Azaheterocyclen;
R^{§}
- OH, -SH, -O-C₁₋₈Alkyl, -O-C₆₋₁₄Aryl, -S-C₁₋₄ Alkyl, -S-C₆₋₁₄Aryl,
- SO-C₁₋₄Alkyl, -SO-C₆₋₁₄Aryl, -SO₂-C₁₋₄Alkyl, -SO₂-C₆₋₁₄Aryl, -SO₃H,
- OSO₂C₁₋₈Alkyl, -OSO₂C₆₋₁₄Aryl, -COOH, -COOC₁₋₈Alkyl, -(CO)C₁₋₈Alkyl,
- COOH, -CONH₂, -CONHC₁₋₆Alkyl, -CON(C₁₋₆Alkyl)₂,
- NH₂, -NHC₁₋₆Alkyl, -N(C₁₋₆Alkyl)₂, -NHC₆₋₁₄Aryl, -NH-Hetaryl,
- N(C₆₋₁₄Aryl)₂, -N(C₁₋₆Alkyl)(C₆₋₁₄Aryl),
- CH₃, -CHF₂, -CF₃, -C₂H₅, -C(CH₃)₂, -(CH₂) ₂CH₃, -(CH₂) ₃CH₃, -CH₂CH₂OH,
- CH₂CH₂SH, -CH₂CH₂SCH₃, -CH₂CF₃, -CH₂CCl₃, -CH₂CHF₂, -CH₂CHCl₂,
- CH₂CH₂F, -CH₂CH₂Cl, -CH₂CH₂Br, -Cylopropyl, -Cylopropylmethyl,
- Cylobutyl, -Cyclobutylmethyl, -Cyclopentyl, -Cyclopentylmethyl,
- Cylohexyl, -Cyclohexylmethyl,
- F, -Cl, -Br, -I, -CN, -NO₂, und -SCN.
R* =O, =S, =N-H, =N-C₁₋₈Alkyl, =N-Aryl, =N-OH, =N-O-C₁₋₈Alkyl, =N-O-C₆₋₁₄ Aryl
sowie pharmazeutisch verträgliche Salze, Solvate, aktive Metabolite, Tautomere und Prodrugs dieser Verbindungen,
wobei die folgenden Verbindungen ausgenommen sind:
- 1,4-Dihydro-2,2-dimethyl-5-(4-morpholinyl)-10-propylthio-2H-pyrano[4",3":4',5']pyrido[3',2':4',5']thieno[3,2-d]pyrimidin-8(9H)-on,
- 10-Buthylthio-1,4-dihydro-2,2-dimethyl-5-(4-morpholinyl)-2H-pyrano[4",3":4',5']pyrido[3',2':4',5']thieno[3,2-d]pyrimidin-8(9H)-on und
- 2-Methyl-5-thiophen-2-yl-1,2,3,4-tetrahydro-11H-7-thia-2,6,9,11-tetraaza-benzo[c]fluorene-8,10-dion.

3. Verbindung nach Anspruch 1 gemäß Formel 1a oder 1b: 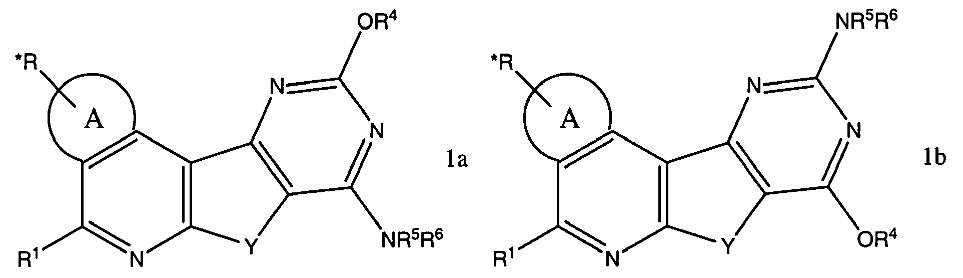
worin R¹, R⁴, R⁵, R⁶ sowie A, R* und Y die oben genannten Bedeutungen besitzen.

4. Verbindung nach Anspruch 1 oder 3, wobei diese aus der Gruppe ausgewählt ist:
10-Ethoxy-5-morpholino-8-piperazin-1-yl-pyrimido[4',5':4,5]thieno[2,3-c]-1,2,3,4-tetrahydro-isochinolin
7-Ethoxy-2,3-dihydro-4-morpholino-9-piperazin-1 yl-1H-cyclopenta[4',5']pyrido[3',2':4,5]thieno[3,2-d]pyrimidin
8-Ethoxy-5-piperidino-10-piperazin-1 -yl-pyrimido[4',5':4,5]thieno[2,3-c]-1,2,3,4-tetrahydro-isochinolin-hydrochlorid
8-Ethoxy-5-phenyl-10-piperazin-1 -yl-pyrimido[4',5':4,5]thieno[2,3-c]-1,2,3,4-tetrahydro-isochinolin-hydrochlorid
10-Ethoxy-5-phenyl-8-piperazin-1-yl-pyrimido[4',5':4,5]thieno[2,3-c]-1,2,3,4-tetrahydro-isochinolin
8-Ethoxy-5-morpholino-10-piperazin-1-yl-pyrimido[4',5':4,5]thieno[2,3-c]-1,2,3,4-tetrahydro-isochinolin
7-Ethoxy-2,3,4,5-tetrahydro-4-morpholino-9-piperazin-1 yl-1 H-cyclohepta[4',5']pyrido[3',2':4,5]thieno[3,2-d]pyrimidin

5. Verbindung nach Anspruch 2 ausgewählt aus:
5-Phenyl-pyrimido[4',5':4,5]thieno[2,3-c]-1,2,3,4-tetrahydro-isochinolin-8,10(9H,11H)-dion

6. Pharmazeutische Zusammensetzung enthaltend mindestens eine der Verbindungen nach einem der Ansprüche 1-5 sowie ggf. pharmazeutisch verträgliche Hilfsstoffe und/oder Träger.

7. Pharmazeutische Zusammensetzung nach Anspruch 6 enthaltend zusätzlich einen oder mehrere der folgenden Wirkstoffe:
- β₂-Adrenoceptor Agonisten
- Dinatriumcromoglycat
- Korticosteroide
- Leukotrien-Antagonisten (entweder Enzym-Inhibitoren [wie 5-Lipoxygenaseinhibitoren oder Arachidonsäure-Enzyminhibitoren] oder Rezeptorantagonisten) ,
- Antihistaminika (bevorzugt solche mit Mastzellen-stabilisierenden Eigenschaften oder Leukotrien-antagonisierenden Aspekten)
- Theophyllin
- Muscarinrezeptor-Antagonisten
- (monoklonale) Antikörper gegen TNF-alpha oder andere Wirkstoffe, die die Bildung bzw. Freisetzung von TNF-alpha oder die Aktivität von TNF-alpha hemmen

8. Verwendung mindestens einer der Verbindungen gemäß einem der Ansprüche 1, 3 und 4 zur Herstellung eines Arzneimittels zur Inhibierung des Enzyms Phosphodiesterase 4 (PDE4) und/oder TNF alpha-Freisetzung.

9. Verwendung mindestens einer der Verbindungen gemäß Anspruch 2 oder 5 zur Herstellung eines Arzneimittels zur Inhibierung der TNF-alpha Freisetzung.

10. Verwendung nach Anspruch 8 oder 9 zur Behandlung von Asthma bronchiale, COPD, Rheumatoide Arthritis (RA), Osteoarthritis, Multiple Sklerose, Guillain-Barré Syndrom, Mb. Crohn, Colitis ulcerosa, Psoriasis, atopische Dermatitis, allergische Ekzeme, Rhinitis allergica, allergische Konjunktivitis, systemische Sklerodermie, Graft versus host disease (GvHD), Systemischer Lupus Erythematodes (SLE), Diabetes mellitus Typ I, neurodegenerative Erkrankungen, insbesondere Mb. Alzheimer und Mb. Parkinson, posttraumatisches Multiorganversagen, Toxisches Schocksyndrom, Akute Glomerulonephritis, akute und chronische Schmerzen, Arteriosklerose, Herzinfarkt, Schlaganfall, Tumorerkrankungen, virale Erkrankungen.
